(19) Europäisches Patentamt
European Patent Office
Office européen des brevets

(11) Veröffentlichungsnummer: **0 013 666**
**A1**

# (12) EUROPÄISCHE PATENTANMELDUNG

(21) Anmeldenummer: 80810010.1

(22) Anmeldetag: 10.01.80

(51) Int. Cl.³: C 07 D 471/04
A 61 K 31/47
//C07D215/26, C07D215/38,
(C07D471/04, 221/00, 221/00)

(30) Priorität: 16.01.79 CH 402/79

(43) Veröffentlichungstag der Anmeldung:
23.07.80 Patentblatt 80/15

(84) Benannte Vertragsstaaten:
AT BE CH DE FR GB IT LU NL SE

(71) Anmelder: CIBA-GEIGY AG
Patentabteilung Postfach
CH-4002 Basel(CH)

(72) Erfinder: Haas, Georges, Dr.
Im Rehwechsel 24
CH-4102 Binningen(CH)

(72) Erfinder: Jaeggi, Knut A., Dr.
General Guisan-Strasse 44
CH-4054 Basel(CH)

(72) Erfinder: Rossi, Alberto, Dr.
Bündtenweg 30
CH4104 Oberwil(CH)

(72) Erfinder: Sele, Alex
Langmattstrasse 14
CH-4132 Muttenz(CH)

## (54) Neue Phenanthrolinderivate, Verfahren zu ihrer Herstellung, diese enthaltende pharmazeutische Präparate und ihre Verwendung.

(57) Neue 4,7-Phenanthrolinderivate der Formel (I),

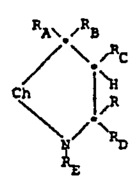

(I),

worin Ch einen gegebenenfalls substituierten 5,6 Chinolinylenrest bedeutet, dessen 6-Stellung mit der Gruppe -N(RE)- verbunden ist, R eine gegebenenfalls veresterte oder amidierte Carboxygruppe bedeutet, und worin entweder RA und RB gemeinsam Oxo darstellen, RC und RD gemeinsam eine zusätzliche Bindung bedeuten und RE einen Rest $R_1$ darstellt, der Wasserstoff oder einen aliphatischen, cycloaliphatischen, araliphatischen oder heterocyclisch-aliphatischen Rest bedeutet, oder RA eine gegebenenfalls verätherte Hydroxygruppe bedeutet und RB gemeinsam mit RC sowie RD gemeinsam mit RE jeweils eine zusätzliche Bindung darstellen, und Salze von salzbildenden Verbindungen der Formel (I),

Verbindungen der Formel (I) oder ihre Salze haben antiallergische Eigenschaften. Sie können nach an sich bekannten Verfahren hergestellt werden und sind als Wirkstoffe von pharmazeutischen Präparaten verwendbar.

EP 0 013 666 A1

Neue Phenanthrolinderivate, Verfahren zu ihrer Herstellung, diese enthaltende pharmazeutische Präparate und ihre Verwendung.

Die Erfindung betrifft neue 4,7-Phenanthrolinderivate der Formel (I)

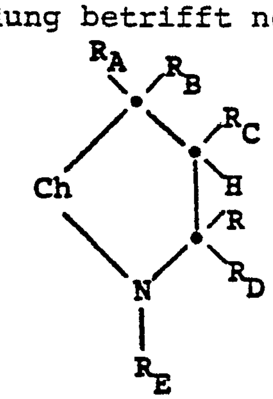

(I),

worin Ch einen gegebenenfalls substituierten 5,6-Chinolinylenrest bedeutet, dessen 6-Stellung mit der Gruppe $-N(R_E)-$ verbunden ist, R eine gegebenenfalls veresterte oder amidierte Carboxygruppe bedeutet, und worin entweder $R_A$ und $R_B$ gemeinsam Oxo darstellen, $R_C$ und $R_D$ gemeinsam eine zusätzliche Bindung bedeuten und $R_E$ einen Rest $R_1$ darstellt, der Wasserstoff oder einen aliphatischen, cycloaliphatischen, araliphatischen oder heterocyclisch-aliphatischen Rest bedeutet, oder $R_A$ eine gegebenenfalls verätherte Hydroxygruppe bedeutet und $R_B$ gemeinsam mit $R_C$

koxy- oder Phenylniederalkoxycarbonyl. Substituiertes Niederalkoxycarbonyl ist z.B. Hydroxy-, Niederalkoxy- oder Diniederalkylamino- bzw. Niederalkylen-, Azaniederalkylen-, Oxaniederalkylen-, oder Thianiederalkylenaminoniederalkoxycarbonyl. Substituenten von Phenylniederalkoxycarbonyl sind z.B. Niederalkyl, Niederalkoxy und/oder Halogen.

In amidiertem Carboxy bedeutet die Aminogruppe beispielsweise gegebenenfalls durch Hydroxy monosubstituiertes oder durch aliphatische Reste mono- oder disubstituiertes Amino, wie Amino, Hydroxyamino, Mono- oder Diniederalkylamino oder Niederalkylenamino bzw. Aza-, Oxa- oder Thianiederalkylenamino mit jeweils 5 bis 7 Ringgliedern.

Veräthertes Hydroxy ist beispielsweise Niederalkoxy, Niederalkenyloxy, Niederalkoxyniederalkoxy oder Diniederalkylamino- bzw. 5- bis 7-gliedriges Niederalkylen- oder Aza-, Oxo- oder Thianiederalkylenaminoniederalkoxy.

Vor- und nachstehend werden unter "niedrigen" organischen Resten und Verbindungen vorzugsweise solche verstanden, die bis und mit 7, vor allem bis und mit 4, Kohlenstoffatome aufweisen.

Niederalkyl bedeutet z.B. Methyl, Aethyl, Propyl, Isopropyl, Butyl, Isobutyl, Secundärbutyl, Tertiärbutyl, Pentyl, Hexyl oder Heptyl.

Niederalkenyl bedeutet z.B. Vinyl, 1-Methylvinyl, 1-Aethylvinyl, Allyl oder 2- oder 3-Methallyl.

Niederalkoxy sowie Niederalkoxy in Niederalkoxycarbonyl bedeutet z.B. Methoxy, Aethoxy, Propyloxy, Isopropyloxy, Butyloxy, Isobutyloxy, Secundärbutyloxy, Tertiärbutyloxy, Pentyloxy, Hexyloxy oder Heptyloxy. Niederalkylthio ist z.B. Methylthio, Aethylthio, Propylthio, Butyl-

sowie $R_D$ gemeinsam mit $R_E$ jeweils eine zusätzliche Bindung darstellen, und Salze von salzbildenden Verbindungen der Formel (I), Verbindungen der Formel (I) oder ihre Salze enthaltende pharmazeutische Präparate und die Verwendung von Verbindungen der Formel (I) und ihrer Salze.

Aliphatische, cycloaliphatische und araliphatische Reste sind in erster Linie gegebenenfalls substituierte aliphatische, cycloaliphatische oder araliphatische Kohlenwasserstoffreste, wie entsprechendes Niederalkyl, Niederalkenyl, Cycloalkyl oder Phenylniederalkyl. Substituenten sind z.B. Hydroxy und/oder Niederalkoxy, ferner Niederalkyl- oder Phenylthio, Niederalkan- oder Benzolsulfinyl oder Niederalkan- oder Benzolsulfonyl oder Diniederalkylamino bzw. Niederalkylen- oder Aza-, Oxa- oder Thianiederalkylenamino mit 5 bis 7 Ringgliedern. Substituierte Kohlenwasserstoffreste dieser Art sind insbesondere Hydroxy-, Niederalkoxy- und Diniederalkylaminoniederalkyl, ferner 5- bis 7-gliedrige Niederalkylenamino-, bzw. Aza-, Oxa- oder Thianiederalkylenaminoniederalkyl. Heterocyclyl in heterocyclischen oder heterocyclisch-aliphatischen Resten ist in erster Linie monocyclisches Heterocyclyl aromatischen Charakters mit einem Heteroatom, wie Sauerstoff, Schwefel oder Stickstoff, als Ringglied, wie Furyl, Thienyl oder Pyridyl. In heterocyclisch-aliphatischen Resten ist der aliphatische Teil z.B. ein entsprechender aliphatischer Kohlenwasserstoffrest, insbesondere Niederalkyl.

Als zusätzliche Substituenten von Ch kommen beispielsweise Niederalkyl, Niederalkoxy, Trifluormethyl, Hydroxy und/oder Halogen in Betracht, wobei ein oder mehr als ein Substituent vorhanden sein kann.

Verestertes Carboxy ist beispielsweise mit einem aliphatischen oder araliphatischen Alkohol, wie einem gegebenenfalls substituierten aliphatischen oder araliphatischen Alkohol, verestertes Carboxy, z.B. entsprechendes Niederal-

Cycloalkyl ist insbesondere Cycloalkyl mit 3 bis 8, vor allem 5 bis 7, Ringgliedern, z.B. Cyclopentyl, Cyclohexyl oder Cycloheptyl, ferner Cyclopropyl, Cyclobutyl, oder Cyclooctyl.

Furylniederalkyl, Thienylniederalkyl, bzw. Pyridylniederalky weist als Alkylteil insbesondere Methyl auf und stellt beispielsweise Furfuryl, 2-Thenyl oder Pyridylmethyl, wie 2- oder 4-Pyridylmethyl, dar.

Mono- oder Diniederalkylamino bedeutet z.B. Methylamino, Dimethylamino, Aethylamino, Diäthylamino, Propylamino oder Butylamino.

Niederalkylenamino bzw. Aza-, Oxa- oder Thianiederalkylenamino sowie solches in entsprechend substituierten Niederalkyl- bzw. Niederalkoxyresten bedeutet z.B. Pyrrolidino, Piperidino, Morpholino, Thiamorpholino, oder N'-Niederalkyl-, wie N'-Methylpiperazino.

Halogen ist insbesondere Halogen bis und mit Atomnummer 35 wie Fluor, Chlor oder Brom.

Salzbildende Verbindungen der Formel (I) sind beispielsweise Carboxy R und/oder enolische Hydroxygruppen, z.B. Hydroxy $R_A$, aufweisende saure Verbindungen oder basische Gruppen aufweisende basische Verbindungen der Formel (I). Salze derselben sind beispielsweise Salze von sauren Verbindungen mit Basen, insbesondere mit pharmazeutisch verwendbaren Basen, wie Alkalimetall- oder Erdalkalimetall-, z.B. Natrium Kalium, Magnesium- oder Calciumsalze, ferner Ammoniumsalze mit Ammoniak oder Aminen, wie Niederalkyl- oder Hydroxyniederalkylaminen, z.B. Trimethylamin, Triäthyl-

thio, Pentylthio, Hexylthio oder Heptylthio.

Hydroxyniederalkyl ist insbesondere 2- und/oder 3-Hydroxyniederalkyl, z.B. 2-Hydroxyäthyl, 3-Hydroxypropyl oder 2,3-Dihydroxypropyl. Entsprechend ist Hydroxyniederalkoxy sowie Hydroxyniederalkoxy in Hydroxyniederalkoxycarbonyl insbesondere 2- und/oder 3-Hydroxyniederalkoxy, z.B. 2-Hydroxyäthoxy,3-Hydroxypropyloxy oder 2,3-Dihydroxypropyloxy.

Niederalkoxyniederalkyl ist insbesondere 2- und/oder 3-Niederalkoxyniederalkyl, z.B. 2-Methoxyäthyl, 2-Aethoxyäthyl oder 3-Methoxypropyl. Entsprechend ist Niederalkoxyniederalkoxy sowie solches in Niederalkoxyniederalkoxycarbonyl insbesondere 2-und/oder 3-Niederalkoxyniederalkoxy z.B. 2-Methoxyäthoxy, 2-Aethoxyäthoxy oder 3-Methoxypropyloxy.

Diniederalkylaminoniederalkyl ist inbesondere 2- und/oder 3-Diniederalkylaminoniederalkyl, z.B. 2-Dimethylaminoäthyl, 2-Diäthylaminoäthyl oder 3-Dimethylaminopropyl. Entsprechend ist Diniederalkylaminoniederalkoxy sowie solches in Diniederalkylaminoniederalkoxycarbonyl insbesondere 2- und/oder 3-Diniederalkylaminoniederalkoxy, z.B. 2-Dimethylaminoäthoxy, 2-Diäthylaminoäthoxy oder oder 3-Dimethylaminopropyloxy. 5- bis 7-gliedrige Nideralkylenamino- bzw. Aza-, Oxa- oder Thianiederalkylenaminoniederalkyl ist z.B. Pyrrolidino-, Piperidino-, Morpholino-, Thiamorpholino- bzw. oder Piperazino- bzw. N'-Niederalkylpiperazinoniederalkyl.

Phenylniederalkyl ist beispielsweise Benzyl oder 1- oder 2-Phenyläthyl und Phenylniederalkoxy sowie solches in Phenylniederalkoxycarbonyl z.B. Benzyloxy oder 1- oder 2-Phenyläthoxy.

Die Erfindung betrifft insbesondere einerseits Verbindungen der Formel (Ia)

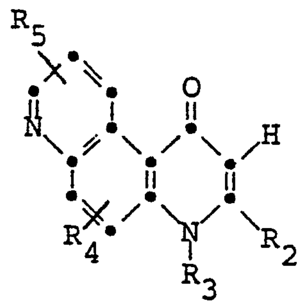

(Ia),

andererseits Verbindungen der Formel (Ib)

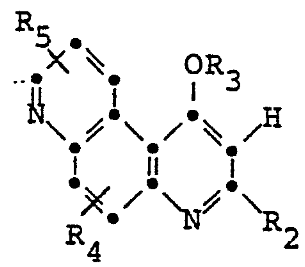

(Ib),

worin $R_2$ für Carboxy, Niederalkoxycarbonyl mit bis zu 5 Kohlenstoffatomen, z.B. Methoxy- oder Aethoxycarbonyl, Hydroxyniederalkoxycarbonyl mit bis zu 5 Kohlenstoffatomen, z.B. 2-Hydroxyäthoxycarbonyl, Niederalkoxyniederalkoxycarbonyl mit bis zu 4 Kohlenstoffatomen in den Alkoxyteilen, z.B. 2-Methoxy- oder 2-Aethoxyäthoxycarbonyl, Diniederalkylaminoniederalkoxycarbonyl mit jeweils bis zu 4 Kohlenstoffatomen im Alkyl- und Alkoxyteil, z.B. 2—Dimethylaminoäthoxy- oder 2-Diäthylaminoäthoxycarbonyl, Carbamyl N-Hydroxycarbamyl oder N-Niederalkyl- bzw. N,N-Diniederalkylcarbamyl mit bis zu 4 Kohlenstoffatomen im Alkyl, z.B. N-Methyl-, N-Aethyl- oder N,N-Dimethylcarbamyl, steht, $R_3$ Wasserstoff, Niederalkyl mit bis zu 4 Kohlenstoffatomen, z.B. Methyl, Niederalkenyl mit bis zu 4 Kohlenstoffatomen, gegebenenfalls durch Niederalkyl mit bis zu 4 Kohlenstoffatomen, z.B. Methyl, Niederalkoxy mit bis zu

4 Kohlenstoffatomen, z.B. Methoxy, und/oder Halogen bis und mit Atomnummer 35, z.B. Chlor, substituiertes Phenyl-, Furyl-, Thienyl- oder Pyridylniederalkyl mit bis zu 4 Kohlenstoffatomen im Alkylteil, z.B. entsprechendes Benzyl, 1- oder 2-Phenyläthyl, Furfuryl, 2-Thenyl oder 2- oder 4-Picolyl, Niederalkoxyniederalkyl mit bis zu 4 Kohlenstoffatomen im Alkoxy- bzw. Alkylteil, z.B. 2-Methoxy- oder 2-Aethoxyäthyl, oder Diniederalkylaminoniederalkyl bzw. Niederalkylen- oder Aza-, Thia-, oder Oxaniederalkylenaminoniederalkyl, worin Niederalkyl bis zu 4 Kohlenstoffatome und Niederalkylen bzw. Aza-, Oxa- oder Thianiederalkylen 5-7 Ringglieder aufweist, z.B. 2-Dimethylamino- oder 2-Diäthylaminoäthyl, 2-Pyrrolidino-, 2-Piperidino-, 2-Morpholino-, 2-Thiamorpholino- oder 2-Piperazino- bzw. 2-(4-Methylpiperazino)-äthyl bedeutet, und $R_4$ und $R_5$ unabhängig voneinander Wasserstoff, Niederalkyl mit bis zu 4 Kohlenstoffatomen, z.B. Methyl, Niederalkoxy mit bis zu 4 Kohlenstoffatomen, z.B. Methoxy, Hydroxy oder Halogen bis einschliesslich Atomnummer 35, z.B. Chlor, bedeuten, und Salze von salzbildenden Verbindungen der Formel Ia bzw. Ib .

Die Erfindung betrifft vor allem einerseits Verbindungen der Formel (II)

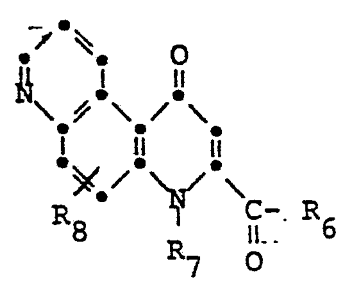

(II),

andererseits Verbindungen der Formel (III) .

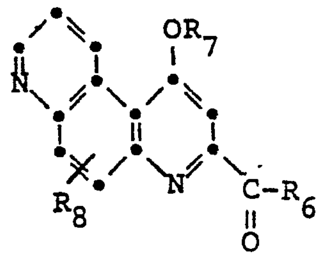

(III),

worin $R_6$ Hydroxy oder vor allem Niederalkoxy mit bis zu 4 Kohlenstoffatomen, z.B. Methoxy, Aethoxy, Propyloxy oder Butyloxy bedeutet, $R_7$ Wasserstoff oder Niederalkyl mit bis zu 4 Kohlenstoffatomen, z.B. Methyl oder Aethyl, bedeutet, und $R_8$ Wasserstoff oder vor allem Niederalkoxy mit bis zu 4 Kohlenstoffatomen, wie Methoxy oder Aethoxy, bedeutet und Salze von salzbildenden Verbindungen der Formel II und III.

Die Erfindung betrifft namentlich die in den Beispielen genannten Verbindungen der Formel (I).

Die Verbindungen der Formel (I) können nach an sich bekannten Methoden hergestellt werden, beispielsweise indem man eine Verbindung der Formel (IV)

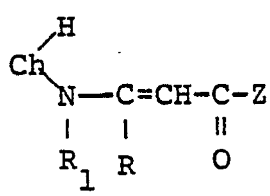

(IV)

worin Z einen abspaltbaren Rest bedeutet, oder ein Salz davon intramolekular cyclisiert und gewünschtenfalls die erhaltene Verbindung in eine andere Verbindung der Formel (I) überführt und/oder eine erhaltene freie Verbindung in ein Salz oder ein erhaltenes Salz in die freie Verbindung oder in ein anderes Salz umwandelt.

Der abspaltbare Rest Z ist beispielsweise eine gegebenenfalls verätherte oder veresterte Hydroxygruppe. Verätherte Hydroxygruppen sind beispielsweise mit aliphatischen, araliphatischen oder aromatischen Alkoholen verätherte Hydroxygruppen, wie Niederalkoxy, z.B. Methoxy oder Aethoxy, oder gegebenenfalls substituiertes wie Niederalkyl, Niederalkoxy oder insbesondere Halogen und/oder Nitro aufweisendes Phenoxy z.B. Phenoxy, 4-Chlorphenoxy, 4-Nitrophenoxy, 2,4-Dinitrophenoxy und 3,5-Dichlorphenoxy. Veresterte Hydroxygruppen sind beispielsweise mit organischen Carbonsäuren, wie Niederalkansäuren, oder mit monofunktionellen Kohlensäurederivaten, wie Kohlensäuremonoestern oder -monohalogeniden, insbesondere aber mit Mineralsäuren wie Halogenwasserstoffsäuren, veresterte Hydroxygruppen, beispielsweise Formyloxy, Acetoxy, Chlorcarbonyloxy, Niederalkoxy-, wie Aethoxycarbonyloxy, oder insbesondere Halogenatome, wie Chlor, Brom oder Jod, ferner Sulfonyloxygruppen, wie von organischen Sulfonsäuren oder Halogensulfonsäuren abgeleitete Sulfonyloxygruppen, z.B. Fluorsulfonyloxy, Chlorsulfonyloxy, Methansulfonyloxy, Benzolsulfonyloxy, p-Toluolsulfonyloxy oder p-Bromsulfonyloxy. Salze von Verbindungen der Formel (IV) sind insbesondere Salze mit Basen, wie Alkalimetallsalze, von Verbindungen der Formel (IV), in denen Z Hydroxy und/oder R Carboxy ist, oder Säureadditionssalze von basischen Verbindungen der Formel (IV).

Die intramolekulare Kondensation kann in üblicher Weise durchgeführt werden, vorzugsweise durch Erhitzen, beispielsweise auf etwa 150° C bis etwa 250° C, erforderlichenfalls in Gegenwart eines unter den Reaktionsbedingungen inerten Lösungsmittels und/oder eines geeigneten Kondensationsmittels, wenn notwendig, unter Inertgas, wie Stickstoff, und/oder im geschlossenen Gefäss.

Unter den Reaktionsbedingungen inerter Lösungsmittel sind beispielsweise höher siedende Kohlenwasserstoffe, wie Toluol oder Xylol, Aether, wie Diphenyläther oder tertiärer Carbonsäureamide, wie Dimethylformamid oder N-Methylpyrrolidon. Geeignete Kondensationsmittel sind beispielsweise saure Mittel, wie Protonensäuren, z.B. Mineralsäuren, u.a. Schwefelsäure, Phosphorsäure oder Polyphosphorsäure, oder saure Mineralsäureester, wie Mono- oder Diniederalkylphosphate oder -phosphite u.a. Triäthylphosphat, Triäthylphosphit oder Tetraäthylpyrophosphat, ferner Lewissäuren, wie beispielsweise Aluminiumchlorid, Aluminiumbromid, Zinkchlorid, Bortrifluorid oder Antimonpentachlorid.

Die Ausgangssstoffe der Formel (IV) können nach an sich bekannten Methoden erhalten werden, beispielsweise ausgehend von entsprechenden 6-Aminochinolinen, zur Herstellung von Ausgangsstoffen der Formel (IV), in denen Z eine verätherte Hydroxygruppe bedeutet, z.B. indem man die 6-Aminochinolinverbindung in üblicher Weise mit einem Acetylendicarbonsäurederivat der Formel R -C≡C-C(=O)-Z (V), z.B. einem Acetylencarbonsäurediester, wie einem Acetylendicarbonsäurediniederalkylester, oder einem anderen geeigneten aliphatischen 1,4-Dicarbonsäurederivat, z.B. mit Chlorfumarsäure oder Oxalessigsäure oder einem Diester, Esterhalogenid oder Dihalogenid davon, kondensiert und gewünschtenfalls einen erhaltenen Ester zur Säure hydrolysiert und/oder eine erhaltene Säure in ein anderes funktionelles Derivat überführt.
So kann man beispielsweise nach Art einer Conrad-Limpach-Reaktion in einem aromatischen bzw. aliphatischen Kohlenwasserstoff, z.B. in Benzol oder Toluol, mit einem Oxalessigsäuremono- oder diniederalkylester umsetzen, wobei man vorzugsweise bei erhöhter Temperatur, z.B bei 50 bis 150°C, vor allem bei 80 bis 110°C, und

vorteilhaft unter azeotrop- destillativer Entfernung des Reaktionswassers arbeitet. Alternativ kann man in bekannter Weise, z.B. in Gegenwart eines basischen Kondensationsmittels, wie wässriger Natron- oder Kalilauge, Pyridin oder Triäthylamin, mit einem Oxalessigsäureesterchlorid oder -monochlorid oder, vorzugsweise in einem Alkanol, wie Methanol, mit einem Acetylendicarbonsäurediniederalkylester, z.B. mit Acetylendicarbonsäuredimethylester, umsetzen.

Die Verbindungen der Formel (I) können ferner hergestellt werden, indem man eine Verbindung der Formel (VI)

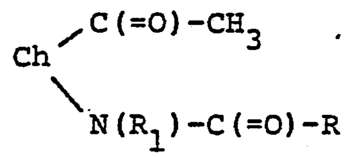

(VI)

oder ein Salz davon intramolekular cyclisiert und gewünschtenfalls die erhaltene Verbindung in eine andere Verbindung der Formel (I) überführt und/oder eine erhaltene freie Verbindung in ein Salz oder ein erhaltenes Salz in die freie Verbindung oder in ein anderes Salz umwandelt.

Die intramolekulare Cyclisierung erfolgt in üblicher Weise, vorzugsweise in einem weitgehend wasserfreien Lösungsmittel, vorteilhaft in Gegenwart eines wasserentziehenden Mittels und erforderlichenfalls in Anwesenheit eines basischen Kondensationsmittels, wenn nötig, bei erhöhter Temperatur, z.B. bei etwa 50 bis 150°C, unter Inertgas, wie Stickstoff, und/oder im geschlossenen Gefäss. Geeignete Lösungsmittel sind vor allem Niederalkanole, wie Methanol, Aethanol oder Butanol, Niederalkylenglykole, wie Aethylenglykol, ferner Dimethylsulfoxid, Dimethylformamid, Diphenyläther und hochsiedenden

Kohlenwasserstoffe, wie Xylole. Basische Kondensationsmittel sind beispielsweise Alkalialkoholate, wie Alkaliniederalkanolate, z.B. Natriummethanolat, Natriumäthanolat oder Natrium- tert.-butanolat, oder Alkalimetallhydride, wie Natriumhydrid.

Die Ausgangsstoffe der Formel (VI) können nach an sich bekannten Methoden hergestellt werden, beispielsweise indem man ein entsprechendes 5-Acetyl-6-aminochinolin mit einer Verbindung der Formel

R-COOH (VII)

oder einem reaktionsfähigen funktionellen Derivat, wie einem Ester, z.B. einem Diniederalkylester, oder Esterhalogenid, z.B. einem Niederalkylesterchlorid, davon umsetzt, und gewünschtenfalls in der erhaltenen Verbindung der Formel (VI) R in üblicher Weise in eine andere Gruppe R überführt.

Die Verbindungen der Formel (I) können ferner hergestellt werden, indem man eine Verbindung der Formel (VIII)

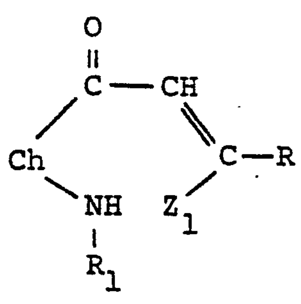

(VIII),

worin $Z_1$ eine gegebenenfalls reaktionsfähige abgewandelte Hydroxygruppe bedeutet, oder ein Tautomeres und/oder Salz davon intramolekular cyclisiert, und gewünschtenfalls eine

so erhältliche Verbindung in eine andere Verbindung der Formel (I) überführt und/oder eine erhaltene freie Verbindung in ein Salz oder ein erhaltenes Salz in die freie Verbindung oder in ein anderes Salz überführt.

Reaktionsfähige abgewandelte Hydroxygruppen sind beispielsweise verätherte oder vor allem veresterte Hydroxygruppen. Verätherte Hydroxygruppen sind z.B. Niederalkoxy oder gegebenenfalls substituiertes Phenoxy und veresterte Hydroxygruppen insbesondere mit Mineralsäuren, wie Halogenwasserstoffsäuren, oder mit Halogensulfonsäuren bzw. organischen Sulfonsäuren veresterte Hydroxygruppen, wie Halogen, z.B. Chlor-, Brom oder Jod, oder Methan-, Benzol-, p-Toluol-, p-Brombenzol-, oder Fluorsulfonyloxy. Die intramolekulare Cyclisierung von Verbindungen der Formel (VIII) kann in üblicher Weise erfolgen, beispielsweise in einem unter den Reaktionsbedingungen inerten Lösungsmittel wie einem Kohlenwasserstoff, z.B. von Benzol, Toluol oder Mineralöl, einem Aether , z.B. Diäthyläther oder Tetrahydrofuran, Dimethylformamid, Dimethylsulfoxid, und dergleichen, erforderlichenfalls unter Erwärmen z.B. auf etwa 50 bis 150° C, vorteilhaft in Gegenwart eines basischen Kondensationsmittels, wie eine Alkalimetallhydroxydes, z.B. von Natrium-oder Kaliumhydroxid, oder einer organischen Stickstoffbase, z.B. von Pyridin oder Triäthylamin, wenn nötig, unter Inertgas, wie Stickstoff, und/oder im geschlossenen Gefäss.

Die Ausgangsstoffe der Formel (VIII) können nach an sich bekannten Methoden hergestellt werden, beispielsweise indem man in üblicher Weise ein entsprechendes 5-Acetyl-6-amino-chinolin mit

einer Verbindung der Formel $Z_1$-C(=O)-R (IX), z.B. einem entsprechenden Oxalsäureesterchlorid oder Oxalsäurediester, oder eine entsprechende 6-Amino-chinolin-5-carbonsäure oder einen Ester davon mit einer Verbindung der Formel $CH_3$-C(=O)-R (X) umsetzt und gewünschtenfalls in der erhaltenen Verbindung die primär gebildete Hydroxygruppe $Z_1$ in eine andere Gruppe $Z_1$ überführt.

Die Verbindungen der Formel (I) können weiterhin hergestellt werden, indem man in einer Verbindung der Formel (XI)

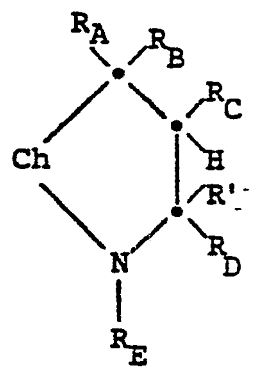

(XI),

worin der Rest R' einen in die gegebenenfalls veresterte oder amidierte Carboxygruppe R überführbaren Rest bedeutet, oder in einem Salz davon die Gruppe R' in die gegebenenfalls veresterte oder amidierte Carboxygruppe R überführt, und gewünschtenfalls eine so erhältliche Verbindung in eine andere Verbindung der Formel (I) oder eine erhaltene salzbildende Verbindung in ein Salz oder ein erhaltenes Salz in die freie Verbindung oder in ein anderes Salz überführt.

In gegebenenfalls verestertes oder amidiertes Carboxy überführbare Gruppen R' sind beispielsweise von verestertem oder amidiertem Carboxy R verschiedene, solvolytisch in gegebenenfalls verestertes oder amidiertes Carboxy überführbaren funktionell abgewandelte Carboxygruppen. Derartige Gruppen sind beispielsweise Cyano, anhydridisierte Carboxygruppen, Iminoäthergruppen, Iminoestergruppen sowie verätherte und/oder veresterte Trihydroxymethylgruppen. Anhydridisierte Carboxygruppen sind beispielsweise mit einer Mineralsäure, wie einer Halogenwasserstoffsäure, mit einer Halogensulfonsäure, wie Fluorsulfonsäure, oder einer organischen Sulfon- oder Carbonsäure, wie einer aliphatischen oder aromatischen Sulfon- oder Carbonsäure, anhydridisierte Carboxygruppen. Iminoäthergruppen sind beispielsweise von veresterten Carboxygruppen R abgeleitete Iminoäthergruppen, wie O-Niederalkylcarbamoyl, oder cyclische Iminoäthergruppen, wie 4,4- oder 5,5-Diniederalkyl-, z.B. 4,4- oder 5,5-Dimethyl-4,5-dihydro-oxazolyl-(2), oder 4,4,6-Triniederalkyl- , z.B. 4,4,6-Trimethyl- 5,6-dihydro-oxazinyl-(2). Iminoestergruppen sind beispielsweise von amidierten Carboxygruppen R abgeleitete, mit Halogenwasserstoffsäuren oder organischen Carbonsäuren, wie Niederalkansäuren, veresterte Iminoestergruppen, wie z.B. Chlorcarbimino oder O-Niederalkanoylcarbamoyl. Verätherte und/oder veresterte Trihydroxymethylgruppen sind beispielsweise veresterten Carboxygruppen R entsprechende verätherte und/oder mit Mineralsäuren, wie Halogenwasserstoffsäuren, veresterte Trihydroxymethylgruppen, z.B. Triniederalkoxymethyl, Niederalkoxy-dihalogenmethyl oder Trihalogenmethyl, insbesondere solche, in denen Halogen Chlor oder Brom darstellt. Weitere solvolytisch in amidierte Carboxygruppen R überführbare Gruppen sind beispielsweise diesen entsprechende dihalogenierte Carbamoylgruppen, d.h. entsprechende Amino-dihalogen-, insbesondere -dichlormethylgruppen.

Die genannten Gruppen können durch übliche Hydrolyse in Carboxygruppen, Iminoäthergruppen sowie verätherte Hydroxy-dihalogenmethyl-, wie Niederalkoxy-dihalogenmethylgruppen , auch in verestertes Carboxy,

Amino-dihalogenmethylgruppen in amidierte Carboxygruppen und Cyano in unsubstituierte Carbamoylgruppen überführt werden. Die Hydrolyse erfolgt in üblicher Weise, beispielsweise in Gegenwart eines sauren oder alkalischen Hydrolysemittels, üblicherweise in Gegenwart eines Lösungs- und/oder Verdünnungsmittels oder eines Gemisches davon, und, wenn notwendig, unter Kühlen oder Erwärmen z.B. in einem Temperaturbereich von etwa 0°C bis etwa 120° C, erforderlichenfalls unter Inertgas, wie Stickstoff, und/oder in geschlossenem Gefäss. Saure Hydrolysemittel sind beispielsweise Mineralsäuren, wie Halogenwasserstoffsäuren, z.B. Salzsäure, oder Sauerstoffsäuren des Schwefels oder Phosphors, wie Schwefel- oder Phosphorsäure, ferner organische Sulfonsäuren, z.B. p-Toluolsulfonsäure oder Methansulfonsäure, oder organische Carbonsäuren, wie Niederalkancarbonsäuren, z.B. Ameisen- oder Essigsäuren. Basische Hydrolysemittel sind beispielsweise Alkalimetallhydroxyde, z.B. Natrium- oder Kaliumhydroxyd, ferner Alkalimetallcarbonate, z.B. Natrium- oder Kaliumcarbonat. Geeignete Lösungs- oder Verdünnungsmittel sind vorzugsweise mit Wasser mischbare Lösungsmittel, wie Niederalkanole, z.B. Aethanol oder Methanol, niedere Ketone, z.B. Aceton, oder tertiärer Alkansäureamide, z.B. Dimethylformamid oder N-Methylpyrrolidon.

Anhydridisierte Carboxygruppen sowie cyclische Iminoäthergruppen können ferner durch Alkoholyse, d.h. Umsetzung mit einem entsprechenden Alkohol, in veresterte Carboxygruppen R überführt werden. Dabei arbeitet man in üblicher Weise, ausgehend von Anhydriden beispielsweise in Gegenwart eines basischen Kondensationsmittels, wie eines Alkalimetallhydroxydes oder -carbonates, z.B. von Natrium- oder Kaliumhydroxyd bzw. entsprechender Carbonate, oder von

organischen Stickstoffbasen,wie Pyridin oder Triäthylamin, und ausgehend von cyclischen Iminoäthern vorzugsweise unter wasserfreien Bedingungen, z.B. in Gegenwart von Chlorwasserstoff, Phosphorsäure, Schwefelsäure oder p-Toluolsulfonsäure, erforderlichenfalls unter Erwärmen, beispielsweise im Temperaturbereich von etwa 0°C bis etwa 150°C, unter Inertgas, wie Stickstoff, und/oder in einem geschlossenen Gefäss.

Anhydridisierte Carboxygruppen können ferner durch Ammono-bzw. Aminolyse, d.h. Umsetzung mit Ammoniak oder einem entsprechenden,mindestens ein Wasserstoffatom aufweisenden Amin, in üblicher Weise in amidierte Carboxygruppen R überführt werden.

Weitere in gegebenenfalls veresterte Carboxygruppen R überführbare Reste R' sind oxydativ in diese überführbare Gruppen, wie die oxydativ in Carboxy überführbare, gegebenenfalls hydratisierte Formylgruppe oder oxydativ in veresterte Carboxygruppen überführbare verätherte Hydroxymethylgruppen.

Die Oxydation kann in an sich bekannter Weise durchgeführt werden, beispielsweise durch Umsetzung mit einer oxydierenden Schwermetallverbindung, vorzugsweise einer Chrom-VI- oder Mangan-VII enthaltenden Verbindung, z.B. mit Chromtrioxyd oder insbesondere Kaliumpermanganat, ferner mit Wismut-III-, Mangan-IV- oder Silber-I- enthaltenden Verbindungen, z.B. mit Wismutoxid , Mangandioxyd oder Silberoxid,oder durch Luftoxydation. Dabei arbeitet man vorteilhafterweise in Gegenwart eines gegenüber den Reaktionsteilnehmern inerten Lösungsmittels, z.B.

von Aceton oder Pyridin, oder eines, vorzugsweise wässrigen Gemisches davon, wenn notwenig, unter Kühlen oder Erwärmen, z.B. im Temperaturbereich von etwa 0°C bis 80°C.

Die Ausgangsstoffe der Formel (XI) können nach an sich bekannten Methoden hergestellt werden.

Verbindungen der Formel (XI), in denen R' Cyano, veräthertes Hydroxymethyl oder Formyl bedeutet, kann man beispielsweise durch intramolekulare Kondensation entsprechender Verbindungen der Formel

$$Ch\left\langle\begin{matrix} H \\ N(R_1)-C(R')=CH-C(=O)-Z \end{matrix}\right. \qquad (XII),$$

erhalten in denen Z eine reaktionsfähige veresterte oder eine verätherte Hydroxygruppe, z.B. Halogen oder Niederalkoxy, wie Methoxy, bedeutet, und worin eine Formylgruppe R' auch in intermediär geschützter, wie acetalysierter oder acylalisierter Form, z.B. als Diniederalkoxy-, Niederalkylen-Dioxy- oder Dihalogenmethyl, vorliegen kann. Die, beispielsweise so erhältlichen, Nitrile der Formel (XI) können durch übliche, z.B. säurekatalysierte, Umsetzung mit den entsprechenden Alkoholen bzw. Aminoalkoholen, in Iminoäther der Formel (XI), beispielsweise mit einem Niederalkanol in offenkettige Iminoäther bzw. mit einem betreffenden Aminoalkanol oder Alkandiol, z.B. mit 4-Amino-2-methyl-pentan-2-ol oder 2-Methyl-pentan-2,4-diol, in cyclische Iminoäther, überführt werden. Eine Trihalogenmethylgruppe R'aufweisende Verbindungen der Formel (XI) können beispielsweise erhalten werden durch übliche Halogenierung einer entsprechenden Methylverbindung,z.B.

mit N-Chlor- oder N-Bromsuccinimid, oder durch Haloformanalogen Abbau entsprechender Alkanoyl-, wie Acetylverbindungen.

Eine gegebenenfalls hydratisierte Formylgruppe R'aufweisende Verbindungen der Formel (XI) können ferner im Verlaufe der Oxydationsreaktion in situ, z.B. aus der Methyl- oder Aminomethylgruppe oder der gegebenenfalls mit einer anorganischen Säure, wie einer Halogenwasserstoffsäure, oder mit einer organischen Carbonsäure, wie einer Niederalkancarbonsäure, veresterten Hydroxymethylgruppe gebildet oder aus einem ihrer Derivate, wie einem Acetal, Acylal oder Imin, z.B. einem Niederalkylen- oder Diniederalkylacetal, einer Dihalogen-, wie Dichlormethylverbindung oder einem gegebenenfalls substituierten Benzylimin, in Freiheit gesetzt werden. Amino- bzw. Niederalkoxydihalogenmethylgruppen werden ebenfalls vorteilhaft in situ durch übliche partielle Ammono- bzw. Aminolyse oder Alkoholyse der entsprechenden Trihalogenmethylverbindung hergestellt.

Die Verbindungen der Formel (I) können weiterhin hergestellt werden, indem man in einer Verbindung der Formel (XIII)

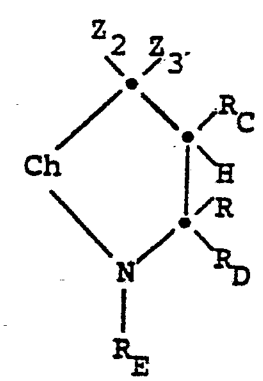

(XIII),

worin $Z_2$ ein in gegebenenfalls veräthertes Hydroxy überführbaren Rest bedeutet und $Z_3$ gemeinsam mit $R_C$ sowie $R_D$ gemeinsam mit $R_E$ jeweils eine zusätzliche Bindung darstellt, oder worin $Z_2$ und $Z_3$ jeweils einen einwertigen oder gemeinsam einen zweiwertigen in Oxo überführbaren

Rest bedeuten, und $R_C$ gemeinsam mit $R_D$ eine zusätzliche Bindung darstellt, oder in einem Salz davon einen in gegebenenfalls veräthertes Hydroxy überführbaren Rest $Z_2$ in gegebenenfalls veräthertes Hydroxy bzw. in Oxo überführbare Reste $Z_2$ und $Z_3$ oder $Z_2 + Z_3$ in Oxo überführt, und gewünschtenfalls die so erhältliche Verbindung in eine andere Verbindung der Formel (I) und/oder eine erhaltene salzbildende Verbindung in ein Salz oder ein erhaltenes Salz in die freie Verbindung oder in ein anderes Salz umwandelt.

In gegebenenfalls veräthertes Hydroxy überführbare Reste sind beispielsweise veresterte Hydroxygruppen, gegebenenfalls verätherte Mercaptogruppen, Sulfinylgruppen, Sulfonylgruppen oder gegebenenfalls substituierte Aminogruppen. Einwertige, gemeinsam in die Oxogruppe überführbare Reste sind beispielsweise veresterte oder mit einem offenkettigen Alkohol verätherte Hydroxygruppen oder mit einem offenkettigen Mercaptan verätherte Mercaptogruppen. Durch $Z_2$ und $Z_3$ gemeinsam dargestellte zweiwertige in Oxo überführbare Reste sind beispielsweise Thioxo, mit einem 2-wertigen Alkohol verätherte Hydroxygruppen, mit einem 2-wertigen Mercaptan verätherten Mercaptogruppen oder gegebenenfalls substituierte Iminogruppen. Veresterte Hydroxygruppen sind beispielsweise mit einer Mineralsäure, einer organischen Sulfonsäure oder einer Carbonsäure veresterte Hydroxygruppen, wie Halogen, z.B. Chlor, Brom oder Jod, aliphatisches oder aromatisches Sulfonyloxy, z.B. Methan-, Aethan-, Benzol- oder p-Toluolsulfonyloxy, oder von einer organischen Carbonsäure oder einem monofunktionellen Kohlensäurederivat, wie einem Kohlensäurehalbester, einer Halogenameisensäure oder der gegebenenfalls substituierte Carbaminsäure, abgeleitetes Acyloxy, z.B. Niederalkanoyloxy, gegebenenfalls substituiertes Benzoyloxy, Niederalkoxycarbonyloxy,

gegebenenfalls substituiertes Phenoxycarbonyloxy, Chlor- oder Bromcarbonyloxy oder gegebenenfalls substituiertes, wie niederalkyliertes, Carbamyloxy. Veräthertes Mercapto ist beispielsweise mit einem aliphatischen oder aromatischen Mercaptan veräthertes Mercapto, z.B. Niederalkylthio oder gegebenenfalls substituiertes Phenylthio. Substituiertes Amino ist beispielsweise durch Hydroxy oder Amino monosubstituiertes, durch aliphatische Reste oder gegebenenfalls substituiertes Phenyl mono- oder disubstituiertes Amino, wobei aliphatische Reste ein- oder zweiwertig sein können, einwertige aliphatische Reste beispielsweise Niederalkyl und zweiwertige aliphatische Reste z.B. Niederalkylen bzw. Aza-, Oxa- oder Thianiederalkylen, darstellen, z.B. Hydroxyamino, Hydrazino, Mono- oder Diniederalkylamino, gegebenenfalls substituiertes Anilino, Pyrrolidino, Piperidino, Morpholino, Thiamorpholino oder N'-Niederalkyl-, wie N'-Methyl-piperazino. Mit einem einwertigen Alkohol veräthertes Hydroxy ist beispielsweise Niederalkoxy oder gegebenenfalls substituiertes Phenyloxy. Mit einem zweiwertigen Alkohol verätherte Hydroxygruppen $Z_2 + Z_3$ sind beispielsweise Niederalkylendioxy- oder gegebenenfalls substituierte 1,2-Phenylendioxy-gruppen, z.B. Aethylendioxy, 1,3-Propylendioxy oder 1,2-Phenylendioxy . Mit einem zweiwertigen Mercaptan verätherte Mercaptogruppen sind beispielsweise Niederalkylendithio- oder gegebenenfalls substituierte 1,2-Phenyldithiogruppen, z.B. Aethylendithio, 1,3-Propylendithio oder 1,2-Phenylendithio. Gegebenenfalls substituierte Iminogruppen sind beispielsweise durch Hydroxy, Amino, Niederalkyl oder gegebenenfalls substituiertes Phenyl substituierte Iminogruppen, z.B. Oximino, Hydrazono, Niederalkylimino oder Anilo.

Die Ueberführung der genannten Gruppen in gegebenenfalls veräthertes Hydroxy bzw. in Oxo erfolgt in üblicher Weise, vorzugsweise durch Solvolyse insbesondere Hydrolyse oder Alkoholyse, d.h. Umsetzung mit Wasser oder dem der zu bildenden verätherten Hydroxygruppe entsprechenden Alkohol. So kann man beispielsweise die genannten in Oxo bzw. Hydroxy überführbaren Gruppen in üblicher Weise, beispielsweise in Gegenwart eines sauren oder basischen Hydrolysemittels, vorteilhaft in einem Lösungs- oder Verdünnungsmittel und erforderlichenfalls bei erhöhter oder erniedrigter Temperatur, z.B. dem Temperaturbereich von 0 - 150° C, unter Inertgas, wie Stickstoff, und/oder in einem geschlossenen Gefäss, in Oxo bzw. Hydroxy überführen. Saure Hydrolysemittel sind beispielsweise Protonensäuren, wie Mineralsäuren oder deren saure Salze, z.B. Chlor-, Brom- oder Iodwasserstoffsäure, Schwefelsäure bzw. Alkalimetallhydrogensulfate, Sulfonsäuren, z.B. p-Toluolsulfonsäure oder Sulfaminsäure, oder organische Carbonsäuren, wie Niederalkansäuren, ferner saure Ionenaustauscher. Basische Kondensationsmittel sind beispielsweise Alkalimetallhydroxyde oder -carbonate z.B. Natrium- oder Kaliumhydroxyd oder -carbonat, ferner tertiäre organische Stickstoffbasen, z.B. Triäthylamin oder Pyridin. Geeignete Lösungs- oder Verdünnungsmittel sind insbesondere mit Wasser mischbare Lösungsmittel, wie Alkohole, z.B. Niederalkanole, cyclische aliphatische Aether, z.B. Dioxan oder Tetrahydrofuran, niederaliphatische Ketone, z.B. Aceton, tertiäre aliphatische Amide oder Lactame, z.B. Dimethylformamid oder N-methylpyrrolidon, oder aliphatische Sulfoxyde, z.B. Dimethylsulfoxyd. Wie bereits erwähnt, können reaktionsfähige veresterte, d.h. mit einer Mineralsäure oder einer organischen Sulfonsäure veresterte, Hydroxygruppen $Z_2$ nach üblichen Alkoholysemethoden in veräthertes Hydroxy überführt werden, beispielsweise in Gegenwart eines basischen Kondensationsmittels, wie eines Alkalimetallhy-

droxydes oder -carbonates, oder indem man den betreffenden Alkohol in Form eines Alkoholates, wie des betreffenden Alkalimetallalkoholates einsetzt, vorzugsweise in einem Lösungs- oder Verdünnungsmittels, erforderlicherweise unter Erwärmen z.B. im Temperaturbereich von etwa 0 - 150° C, und ein Inertgas, wie Stickstoff, und/oder im geschlossenen Gefäss.

Die Ausgangsstoffe der Formel XIII können, nach an sich bekannten Methoden hergestellt werden.

So kann man Verbindungen der Formel XIII, in denen $Z_2$ Halogen oder eine gegebenenfalls substituierte Aminogruppe bedeutet, beispielsweise erhalten, indem man eine Verbindung der Formel XIV

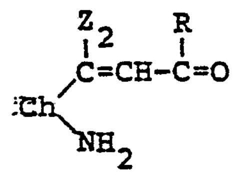

(XIV),

in üblicher Weise ringschliesst.

Aus den z.B. so erhältlichen Halogen-Verbindungen können durch Umsetzung mit einem Isothiuroniumsalz und anschliessende Hydrolyse bzw. Umsetzung mit Natriumthiolacetat und anschliessender Reduktion Verbindungen der Formel XIII, in denen $Z_2$ Mercapto ist, bzw. durch Umsetzung mit einem Alkalimetallmercaptid Verbindungen der Formel XIII, in den $Z_2$ veräthertes Mercapto ist, erhalten werden. Verbindungen der Formel XIII, in denen $Z_2$ eine disubstituierte Aminogruppe oder in denen $Z_2$ und $Z_3$ veräthertes Hydroxy oder Mercapto bedeuten, kann man ferner herstellen, indem man eine Verbindung der Formel XV

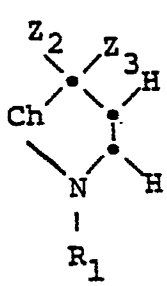

(XV)

durch Umsetzung mit einem metallierenden Mittel, z.B. Phenylnatrium oder Butyllithium, in 2-Stellung zur Gruppe $-N(R_4)-$ metalliert und anschliessend mit einer Verbindung der Formel R-Hal (XVI) oder mit Kohlendioxid umsetzt. Verbindungen der Formel XIII, in denen $Z_2$ + $Z_3$ Imino darstellt, können z.B. erhalten werden durch Kondensation eines entsprechenden 6-Amino-chinolin mit einer Chlorfumarsäureesternitril bzw. mit Acetylendicarbonsäuredinitril, wobei man vorzugsweise in der für die Herstellung und intramolekulare Kondensation von Verbindungen der Formel IV bzw. VI angegebenen Weise arbeitet.

Die Verbindungen der Formel I kann man weiterhin erhalten, indem man in einer Verbindung der Formel XVI

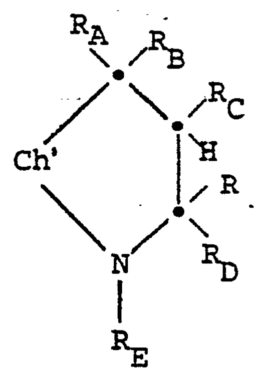

(XVI)

worin Ch' einen partiell hydrierten Rest Ch bedeutet, oder in einem Salz davon den Rest Ch' zu Ch oxydiert, und gewünschtenfalls die so erhältliche Verbindung in eine andere Verbindung der Formel I und/oder eine erhaltene salzbildende Verbindung in ein Salz oder ein erhaltenes Salz in die freie Verbindung oder in ein anderes Salz umwandelt.

Der Rest Ch' ist beispielsweise ein gegebenenfalls zusätzlich wie für den Rest Ch angegeben substituierter 1,2-Dihydro-5,6-chinolinylenrest.

Die Oxydation des Restes Ch' zu dem gewünschten Rest Ch kann in an sich bekannter Weise durchgeführt werden, z.B.durch

Einwirkung eines geeigneten Oxydationsmittels, erforderlichenfalls in Gegenwart eines inerten Lösungsmittels, bei erhöhter Temperatur, z.B. bei etwa 100 bis etwa 300°C, vor allem bei etwa 150 bis etwa 250°C, und/oder in einem geschlossenen Gefäss. Geeignete Oxidationsmittel sind beispielsweise aromatische Nitroverbindungen, wie Nitrobenzol, 2,4-Dinitrochlorbenzol oder Pikrinsäure, anorganische Oxidationsmittel, wie Sauerstoffsäuren des 6-wertigen Schwefels oder des 5-wertigen Phosphors oder Arsens oder deren Anhydride, wie Schwefelsäure, Phosphorpentoxid oder Arsenpentoxid, Metalloxide, wie Eisen-III-oxid, oder organische Carbonylverbindungen, wie Chinone oder, vor allem α,β-ungesättigte, aliphatische Aldehyde und Ketone.

Die Ausgangsstoffe der Formel (XVI) werden vorteilhaft <u>in situ</u> hergestellt, beispielsweise indem man ein entsprechendes 6-Aminochinolin, z.B. der Formel

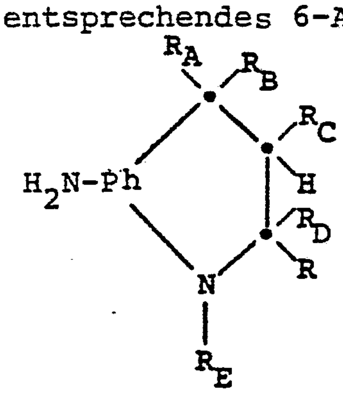

(XVII)

worin Ph einen die Aminogruppe in p-Stellung zur Gruppe $-N(R_E)-$ tragenden 1,2-Phenylenrest bedeutet, oder ein Säureadditionssalz mit einem entsprechenden α,β-ungesättigten aliphatischen Aldehyd oder Keton, z.B. mit Acrolein, in Gegenwart eines sauren Kondensationsmittels, wie einer Lewissäure, z.B. von Aluminiumtrichlorid oder Zinkchlorid, oder einer Mineralsäure, wie Chlorwasserstoffsäure, Schwefelsäure oder Phosphorsäure, bzw. einem Anhydrid einer solchen, z.B. von Phosphorpentoxid, kondensiert. Die als Ausgangsstoffe zu verwendenden α,β-ungesättigten Aldehyde oder Ketone werden vorteilhaft

ebenfalls in situ erzeugt, z.B. durch Autokondensation eines gesättigten aliphatischen Aldehydes, wie Acetaldehyd, oder Ketons, wie Acetons, oder zur in-situ-Bildung von Acrolein durch Dehydratisierung von Glycerin oder dessen Estern.

Verbindungen der Formel I, in denen $R_A$+$R_B$ Oxo, $R_C$+$R_D$ eine zusätzliche Bindung und $R_E$ Wasserstoff bedeuten, können weiterhin hergestellt werden, indem man aus einer Verbindung der Formel XVIII

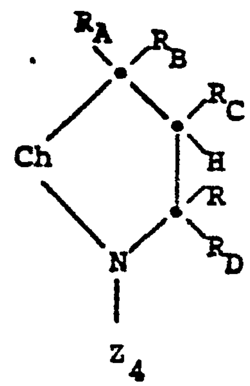

(XVIII),

worin $Z_4$ einen abspaltbaren Rest bedeutet, oder aus einem Salz davon $Z_4$ abspaltet, und gewünschtenfalls die so erhältliche Verbindung in eine andere Verbindung der Formel I und/oder eine erhaltene salzbildende Verbindung in ein Salz oder ein erhaltenes Salz in die freie Verbindung oder in ein anderes Salz überführt.

Abspaltbare Reste sind beispielsweise Acylreste, wie von Carbonsäuren, z.B. organischen Carbonsäuren oder monofunktionellen Derivaten der Kohlensäure, abgeleitete Acylgruppen oder im Phenylteil gegebenenfalls substituierte Alpha-phenylniederalkylgruppen. Acylreste organischer Carbonsäuren sind z.B. gegebenenfalls halogenierte Niederalkanoylreste, wie Acetyl, Propionyl, Butyryl oder Trifluoracetyl , oder gegebenenfalls substituierte Benzoylreste, wie Benzoyl, 3,5-Dichlorbenzoyl oder 4-Nitro- bzw. 2,4-Dinitrobenzoyl. Von monofunktionellen Kohlensäurenderivaten abgeleitete Acylgruppen sind beispielsweise veresterte Carboxygruppen, Halogencarbonylgruppen oder gegebenenfalls substituierte Carbamylgruppen. Veresterte Carboxylgruppen sind beispielsweise gegebenenfalls halogenierte Niederalkoxycarbonylgruppen, wie Niederalkoxycarbonyl z.B. Methoxycarbonyl, Aethoxycarbonyl oder Tertiärbutyloxycarbonyl, Mono-, Di- oder Trihalogen-niederalkoxycarbonylgruppen, z.B. 2-Iodaethoxy-,2,2,2-Tribromaethoxy- oder 2,2,2-Trichloraethoxycarbonyl, gegebenenfalls substituierte α-Phenylniederalkoxycarbonylreste,z.B. Carbobenzoxy, gegebenenfalls substituiertes Phenoxycarbonyl, 2-Sulphonylniederalkoxycarbonylreste, z.B. 2-(4-Toluolsulfonyl)-aethoxycarbonyl, oder verätherte 2-Mercaptoniederalkoxycarbonylreste,z.B. 2-(4-Tolylthio)-äthoxycarbonyl. Substituiertes Carbamyl ist beispielsweise durch Niederalkyl oder Niederalkylen bzw. Aza-, Oxa- oder Thianiederalkylen substituiertes Carbamyl, z.B. N,N-Dimethylcarbamyl, Pyrrolidinocarbonyl, Piperidinocarbonyl, Morpholinocarbonyl, Thiamorpholinocarbonyl oder N'-Niederalkylpiperazinocarbonyl. Im Phenylteil gegebenenfalls substituierte α-Phenylniederalkylreste sind insbesondere entsprechende Benzylreste.

Die Abspaltung der genannten Reste erfolgt beispielsweise durch Solvolyse, insbesondere Hydrolyse, erforderlichenfalls in Gegenwart eines sauren oder basischen Hydrolysemittels, in einem geeigneten Lösungs- oder Verdünnungsmittel, unter Kühlen oder Erwärmen, z.B. im Temperaturbereich von etwa 0 - 150° C, unter Inertgas,wie Stickstoff und/oder im geschlossenen Gefäss. Saure Hydrolysemittel sind beispielsweise Protonensäuren, wie Mineralsäuren, z.B. Chlor-, Brom- oder Iodwasserstoffsäure, Schwefelsäure oder Phosphorsäure, Sulfonsäuren, z.B. Methan-, Aethan-, Benzol- oder p-Toluolsulfonsäure, ferner Sulfaminsäure, oder organische Carbonsäuren, wie Niederalkansäuren, z.B. Essigsäure oder Ameisensäure. Basische Hydrolysemittel sind beispielsweise Alkalimetallhydroxyde oder -carbonate, z.B. Natriumhydroxyd bzw. -carbonat oder Kaliumhydroxyd

bzw. -carbonat, Ammoniak oder Amine. Geeignete Lösungsmittel sind insbesondere mit Wasser mischbare Lösungsmittel. Die solvolytische Abspaltung, z.B. von Acyl, kann ferner durch Ammonolyse bzw. Aminolyse erfolgen, beispielsweise durch Umsetzung des Ammoniak oder einem Amin, wie Hydrazin oder einem Mono- oder Di-niederalkylamin oder Alkylen- bzw. Aza-, Oxa- oder Thiaalkylenamin, z.B. mit Ammoniak, Hydrazin, Methyl- oder Dimethylamin, Morpholin oder Piperidin, erforderlichenfalls in einem inerten Lösungsmittel, unter Kühlen oder Erwärmen, z.B. im Temperaturbereich von etwa -20 - 100°C, und/oder im geschlossenen Gefäss.

Gegebenenfalls substituierte α-Phenylniederalkoxycarbonylgruppen sowie geeignet halogenierte Niederalkoxycarbonylreste können ferner reduktiv abgespalten werden. Die Abspaltung erfolgt in üblicher Weise, insbesondere wie für analoge Abspaltungsreaktionen aus der Literatur bekannt. Ausgehend von gegebenenfalls substituierten Alphaphenylniederalkoxycarbonylreste lässt man beispielsweise auf die zu reduzierende Verbindung in Gegenwart eines Hydrierungskatalysators, wie eines Palladium-, Platin- oder Nickelkatalysators, z.B. von gegebenenfalls sulfidiertem Palladium auf Kohle, von Platin, gegebenenfalls auf Kaliumcarbonat, oder von Raneynickel, Wasserstoff einwirken, erforderlichenfalls in einem geeigneten Lösungsmittel, bei normalem oder erhöhtem Druck, z.B. bei etwa 0,5 bis 20 bar und/oder unter Kühlen oder Erwärmen, z.B. im Temperaturbereich von etwa 0 - 80° C, vorteilhaft in einem geschlossenen Gefäss. 2-Halogenniederalkoxycarbonylreste, wie 2,2,2-Trichloraethoxycarbonyl oder 2-Iodaethoxycarbonyl, können insbesondere durch metallische Reduktion, z.B. durch Behandeln mit unedlen Metallen oder Metall-Legierungen, wie Amalgamen, insbesondere mit Zink, ferner mit aktiviertem, wie mit einer Quecksilbersalzlösung behandelten, Aluminium, üblicherweise in Gegenwart einer Protonensäure, wie einer organischen Carbonsäure, z.B. von Essigsäure, erforderlichenfalls unter Kühlen oder Erwärmen, z.B. bei -30 - +100°C reduziert werden. 2-Halogenniederalkoxycarbonylreste können ferner durch Einwirkung von Chrom-II-Verbindungen, z.B. von Crom-II-clorid oder Chrom-II-acetat reduziert werden.

Die Ausgangsstoffe der Formel XVIII können nach an sich bekannten Methoden hergestellt werden, beispielsweise durch Ringschluss einer Verbindung der Formel H-C -($NZ_4$)-CR=CH-COOH (XIX) oder eines geeigneten funktionellen Derivates, wie eines Esters oder Anhydrides, davon erfolgen, insbesondere in Analogie zu der für die intramolekulare Kondensation von Verbindungen der Formel IV beschriebenen Weise.

Eine erfindungsgemäss erhältliche Verbindung der Formel I kann in an sich bekannter Weise in eine andere Verbindung der Formel I umgewandelt werden.

So kann man in einer Verbindung der Formel I Carboxy R nach an sich bekannten Veresterungsverfahren in eine veresterte Carboxygruppe umwandeln. So kann man z.B. eine Carbonsäure der Formel I in Gegenwart eines geeigneten Kondensationsmittels, wie eines dehydratisierenden Mittels, z.B. von Dicyclohexylcarbodiimid, oder einer Mineralsäure, z.B. von Schwefelsäure oder Chlorwasserstoffsäure, mit dem entsprechenden Alkohol oder, zur Bildung einer Hydroxyniederalkylgruppe, mit einem entsprechenden Epoxyniederalkan umsetzen. Die Veresterung kann man ferner durch Behandeln mit einem geeigneten N,N-Diniederalkylformamidacetal, z.B. N,N-Dimethylformamiddiäthylacetal, oder N,N,O-Trimethylformamidiniummethosulfat, einem Carbonat oder Pyrocarbonat, z.B. mit Diäthyl(pyro)carbonat, oder mit organischem Sulfit oder Phosphit, wie einem Diniederalkylsulfit oder Triniederalkylphosphit, letzteres in Gegenwart eines geeigneten sauren Mittels, z.B. von p-Toluolsulfonsäure, durchführen. Ferner kann man eine zu veresternde Säure der Formel I, worin die freie Carboxygruppe in Salzform, z.B. in einer Alkalimetallsalz-, wie der Natriumsalzform, vorliegt, mit einem reaktionsfähigen Ester eines Alkohols, z.B. mit einer starken Säure, wie einem entsprechenden Halogenid, z.B. Chlorid, Bromid oder Iodid, oder mit Schwefelsäureester umsetzen. Die Veresterung kann man jedoch auch durch Umsetzung mit einer einzuführenden Niederalkoxygruppe entsprechenden Niederalken, vorzugsweise in Gegenwart eines sauren Kondensationsmittels, wie einer starken Protonensäure, z.B. von Schwefelsäure oder einer Lewissäure, z.B. von Bortrifluorid-ätherat, durchführen. In einem der genannten Veresterungsreagentien können gegebenenfalls vorhandene Substituenten in funktionell abgewandelter Form vorliegen und dann in einer Verbindung der Formel I, worin R für substituiertes Niederalkoxycarbonyl steht, in welcher Substituenten in funktionell abgewandelter Form vorliegen, freigesetzt werden. So kann man als Veresterungsreagenz z.B. das 2,3-Epoxy-propylchlorid verwenden und im erhaltenen Ester die 2,3-Epoxy-propyloxy-gruppierung nachträglich zur gewünschten 2,3-Dihydroxy-propyloxygruppierung hydrolisieren.

In Verbindungen der Formel I kann ferner verestertes Carboxy R durch Umesterung, z.B. durch Behandeln mit einem Alkohol, erforderlichenfalls in Gegenwart eines geeigneten Umesterungskatalysators, wie eines betreffenden Alkalimetall-, z.B. des betreffenden Natrium- oder Kaliumalkoholates, oder einer Mineralsäure, z.B. von Schwefelsäure oder Chlorwasserstoffsäure, in eine andere veresterte Carboxygruppe umgewandelt werden.

In Verbindungen der Formel I kann weiterhin freies oder verestertes Carboxy R in an sich bekannter

Weise in gegebenenfalls substituiertes Carbamyl umgewandelt werden. So kann man z.B. einen Ester der Formel I mit Ammoniak, Hydroxylamin oder einem entsprechenden primären oder sekundären Amin behandeln und so zu Amiden der Formel I gelangen. Ferner kann man das Ammoniumsalz oder ein Aminsalz einer Säure der Formel I durch Dehydratisierung, z.B. unter Erhitzen oder durch Einwirkung eines geeigneten Hydratisierungsmittels, wie Schwefelsäure oder N,N-Dicyclohexylcarbodiimid, in ein Amid der Formel I überführen.

Die Ueberführung von Carboxy R in verestertes oder amidiertes Carboxy kann aber durch reaktionsfähige Abwandlung der Carboxygruppe, z.B. Anhydridisierung durch Umsetzung mit einem Säureanhydrid, z.B. mit Thionylchlorid, Phosphorpentachlorid oder einen Niederalkansäureanhydrid, wie Acetanhydrid, Umwandlung in einen reaktiven Ester, z.B. durch Umsetzung mit 4-Nitrophenol, oder in ein reaktives Amid, z.B. durch Umsetzung mit N,N'-Bis-(1-Imidazoyl)-harnstoff, und anschliessende Umsetzung mit dem betreffenden Alkohol, bzw. mit Ammoniak oder dem betreffenden, mindestens ein Wasserstoffatom aufweisenden Amin erfolgen.

In Verbindungen der Formel I kann man eine veresterte oder amidierte Carboxygruppe R in üblicher Weise in die freie Carboxygruppe überführen, beispielsweise durch Hydrolyse, üblicherweise in Gegenwart eines sauren oder basischen Hydrolysemittels, wie einer Mineral- oder Carbonsäure, z.B. von Chlorwasserstoffsäure, Schwefelsäure oder Essigsäure, oder eines Alkalimetallhydroxydes oder -carbonates, z.B. von Natriumhydroxyd bzw. -carbonat oder von Kaliumhydroxyd bzw. -carbonat.

Ferner kann man in Verbindungen der Formel I, worin $R_A$ und $R_B$ gemeinsam Oxo, $R_C$ und $R_D$ gemeinsam eine zusätzliche Bindung und $R_E$ Wasserstoff bzw. $R_A$ Hydroxy ist und $R_B$ und $R_C$ sowie $R_D$ und $R_E$ gemeinsam je eine zusätzliche Bindung bedeuten, in

üblicher Weise am Stickstoffatom durch einen von Wasserstoff verschiedenen Rest $R_1$ substituieren bzw.am 4-ständigen Sauerstoffatom veräthern.Die Substitution des Ringstickstoffatoms durch $R_1$ bzw. die Verätherung einer ringgebundenen Hydroxygruppe $R_A$ erfolgt in üblicher Weise, beispielsweise durch Umsetzung mit einem reaktionsfähigen Ester eines entsprechenden Alkohols, erforderlichenfalls in Gegenwart eines basischen Kondensationsmittels, wie eines Alkalimetallhydrides, -amides, -alkoholates oder -hydroxydes, z.B. von Natriumhydrid, Natriumamid, Lithium-diisopropylamid, Natriummethanolat, Kaliumtertiärbutanolat oder Kaliumhydroxyd, unter Kühlen oder Erwärmen, z.B. im Temperaturbereich von etwa 0 - 120°C, unter Inertgas, wie Stickstoff und/oder im geschlossenen Gefäss. Reaktionsfähige Ester entsprechender Alkohole sind insbesondere Mineralsäureester, wie Halogenwasserstoffsäure- oder Schwefelsäureester, oder organische Sulfonsäureester, z.B. Methan-, Aethan-, Benzol-, p-Toluol- oder Fluorsulfonsäureester. Bei der Behandlung eines Esters der Formel I mit einem Alkylhalogenid in Gegenwart von Natriumhydrid in Dimethylformamid werden dabei überwiegend am 4-ständigen Sauerstoffatom alkylierte Derivate erhalten.

Ferner kann man in Verbindungen der Formel I zusätzliche Substituenten in den Rest Ch einführen, vorzugsweise Niederalkylgruppen und/oder Halogen. So kann man beispielsweise durch Umsetzung mit einem Niederalkylhalogenid, einem Niederalkanol oder einem Niederalken in Gegenwart einer Lewissäure, z.B. von Aluminiumtrichlorid, Niederalkyl oder in üblicher Weise, z.B. durch Umsetzung mit dem betreffenden Halogen in Gegenwart einer Lewissäure, wie des entsprechenden Eisenhalogenides, das auch <u>in situ</u> aus fein verteiltem Eisen und dem betreffenden Halogen gebildet werden kann, oder durch Behandlung mit N-Chlorsuccinimid, Halogen einführen.

Ferner kann man Hydroxy als Substituenten von Ch in üblicher Weise in Niederalkoxy überführen, z.B. durch Umsetzung mit einem reaktiven Ester eines Niederalkanol, wie einem Niederalkylhalogenid oder Diniederalkylsulfat, vorteilhaft in Gegenwart eines basischen Kondensationsmittels, wie eines Alkalimetallhydroxides oder -carbonates, z.B. von Kaliumcarbonat in Amylalkohol oder Aceton.

Salzbildende Verbindungen der Formel I können in an sich bekannter Weise in Salze überführt werden, beispielsweise durch Behandeln mit einer Base oder einer Säure, üblicherweise in Gegenwart eines Lösungs- oder eines Verdünnungsmittels. Erhaltene Salze können in an sich bekannter Weise in die freien Verbindungen umgewandelt werden, z.B.durch Behandeln mit einem sauren Reagenz , wie einer Mineralsäure, bzw. einem basischen Reagenz, wie einem Alkalimetallhydroxyd. Die Verbindungen der Formel I, einschliesslich ihrer Salze, können auch in Form ihrer Hydrate erhalten werden oder das zur Kristallisation verwendete Lösungsmittel einschliessen. Infolge der engen Beziehung der Verbindungen der Formel I in freier Form und in Form ihrer Salze, sind vor- und nachstehend unter den freien Verbindungen oder ihrer Salze sinn- und zweckgemäss, gegebenenfalls auch die entsprechenden Salze bzw. freien Verbindungen zu verstehen.

Die Erfindung betrifft auch diejenigen Ausführungsformen des Verfahrens, bei denen man von einem ~~auf~~ einer beliebigen Verfahrensstufe als Zwischenprodukte erhältlichen Produkt ausgeht und die fehlenden Verfahrensschritte ausführt oder einen Ausgangsstoff unter den Reaktionsbedingungen bildet oder in Form eines Derivates oder gegebenenfalls eines Salzes verwendet. Beim Verfahren der vorliegenden Erfindung werden vorzugsweise diejenigen Ausgangsstoffe verwendet, welche zu den eingangs als besonders wertvoll

hervorgehobenen Verbindungen führen. Neue Ausgangsstoffe, Analogieverfahren zu ihrer Herstellung und ihre Verwendung bilden ebenfalls einen Gegenstand der vorliegenden Erfindung.

Die Verbindungen der Formel I und ihre Salze zeigen wertvolle pharmakologische Eigenschaften. Insbesondere weisen sie antiallergische Wirkungen auf, die z.B. an der Ratte in Dosen ab etwa 1 mg/kg bei intravenöser und in Dosen ab etwa 10 mg/kg bei oraler Verabreichung in passiven kutanen Anaphylaxietest (PCA-Reaktion), der analog der von Goose und Blair, Immunology, Vol. 16, S. 794 (1969) beschriebenen Methode durchgeführt wird, nachgewiesen werden können. Die passive kutane Anaphylaxie wird dabei nach dem von Ovary, Progr. Allergy, Vol. 5, S. 459 (1958), beschriebenen Verfahren erzeugt. Die antiallergische, insbesondere degranulationshemmende Wirkung der Verbindungen der Formel I und ihrer Salze kann auch <u>in vitro</u> anhand der Histaminfreisetzung aus Peritonealzellen der Ratte im Dosisbereich von etwa 0,1 bis etwa 100 mg/l bei immunologisch induzierter Freisetzung, wobei z.B. mit Nippostrongilus brasiliensis infestierte Ratten verwendet werden, und von etwa 1,0 bis etwa 100 mg/l bei chemisch induzierter Freisetzung, wobei diese z.B. mit einem Polymeren von N-4-Methoxy-phenylethyl-N-methyl-amin bewirkt wird, festgestellt werden. Die Verbindungen der vorliegenden Erfindung sind dementsprechend als Hemmer allergischer Reaktionen, z.B. in der Behandlung und Prophylaxe von allergischen Erkrankungen, wie des Asthma bronchiale, sowohl der "extrinsic" als auch der "instrinsic" Form, oder anderer allergischer Erkrankungen, wie der allergischen Rhinitis, z.B. des Heufiebers, der Konjunktivitis oder allergischer Dermatiden z.B. der Urticaria oder von Ekzemen, verwendbar.

Wie bereits erwähnt, betrifft die vorliegende Erfindung auch pharmazeutische Präparate, welche Verbindungen der Formel I oder pharmazeutisch verwendbare Salze derselben enthalten. Bei den erfindungsgemässen pharmazeutischen Präparaten handelt es sich um solche zur enteralen, wie oralen, nasalen oder rektalen, sowie parenteralen oder buccalen Verabreichung an Warmblüter, welche den pharmakologischen Wirkstoff alleine oder zusammen mit einem pharmazeutisch verwendbaren Trägermaterial enthalten. Die Dosierung des Wirkstoffs hängt von der Warmblüterspezies, dem Alter und dem individuellen Zustand und von der Applikationsweise ab.

Die erfindungsgemässen pharmazeutischen Präparate enthalten z.B. bis etwa 95 %, vorzugsweise von etwa 5 % bis etwa 90 % des Wirkstoffs. Erfindungsgemässe pharmazeutische Präparate sind z.B. solche in Dosiseinheitsformen, wie Dragées, Tabletten, Kapseln, Suppositorien oder Ampullen, ferner Inhalationspräparate bzw. topisch oder lokal, z.B. zur Insufflation, verwendbare pharmazeutische Zubereitungen.

Die pharmazeutischen Präparate der vorliegenden Erfindung werden in an sich bekannter Weise, z.B. mittels konventioneller Misch-, Granulier-, Lösungs- oder Lyophilisierungsverfahren hergestellt. So kann man pharmazeutische Präparate zur oralen Anwendung erhalten, indem man den Wirkstoff mit festen Trägerstoffen kombiniert, das so erhaltene Gemisch gegebenenfalls granuliert und das Gemisch bzw. Granulat, wenn erwünscht oder notwendig, nach Zugabe von geeigneten Hilfsstoffen, zu Tabletten oder Dragée-Kernen verarbeiten. Geeignete Trägerstoffe sind insbesondere Füllstoffe, wie Zucker z.B. Laktose, Saccharose, Mannit oder Sorbit, Cellulosepräparate und/oder Calciumphosphate z.B. Tricalciumphosphat oder Calciumhydrogenphosphat, ferner Bindemittel, wie Stärkekleister, z.B. Mais-, Weizen-, Reis- oder Kartoffelstärkekleister, Gelatine, Tragakant, Methylcellulose und/oder Polyvinylpyrrolidon, und/oder, wenn erwünscht, Sprengmittel, wie die oben genannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglycol. Dragée-Kerne werden mit geeigneten, gegebenenfalls Magensaft-resistenten Ueberzügen versehen, wozu man z.B. konzentrierte Zuckerlösungsn, welche gegebenenfalls arabischen Gummi, Talk, Polyvinylpyrrolidon, Polyäthylenglycol und/oder Titandioxyd enthalten, Lacklösung in geeigneten organischen Lösungsmitteln oder Lösungsmittelgemischen oder, zur Herstellung von Magensaft-resistenten Ueberzügen, Lösung von geeigneten Cellulosepräparaten, wie Acetylcellulosephthalat oder Hydroxypropylmethylcellulosephthalat, verwendet. Den Tabletten- oder Dragéeüberzügen können Farbstoffe oder Pigmente z.B. zur Identifizierung oder zur Kennzeichnung verschiedener Wirkstoffdosen beigefügt werden. Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Laktose, Bindemittel, wie Stärken und/oder Gleitmitteln, wie Talk oder Magnesiumstearat, und gegebenenfalls von Stabilisatoren enthalten. In weichen Kapseln ist der Wirkstoff vorzugsweise in geeigneten Flüssigkeiten, wie fetten Oelen, Paraffinoel oder flüssigen Polyäthylenglycolen, gelöst oder suspendiert, wobei ebenfalls Stabilisatoren hinzugefügt sein können.

Als rektal anwendbare pharmazeutische Präparate kommen z.B. Suppositorien in Betracht, welche aus einer Kombination des Wirkstoffes mit einer Suppositoriengrundmasse bestehen. Als Suppositoriengrundmasse eignen sich z.B. natürliche oder synthetische Triglyceride, Paraffinkohlenwasserstoffe, Polyäthylenglycole oder höhere Alkanole. Ferner können auch Gelatine-Rektalkapseln verwendet werden, die eine Kombination des Wirkstoffs mit einer Grundmasse enthalten. Als Grundmasse kommen z.B. flüssige Triglyceride, Polyethylenglycole oder Paraffinkohlenwasserstoffe in Frage.

Zur parenteralen Verabreichung eignen sich in erster Linie wässrige Lösungen des Wirkstoffs in wasserlöslicher Form, z.B. eines wasserlöslichen Salzes, ferner Suspensionen des Wirkstoffs, wie entsprechende oelige Injektionssuspensionen, wobei man geeignete lipophile Lösungsmittel oder Vehikel, wie fette Oele, z.B. Sesamoel oder synthetische Fettsäureester, z.B. Aethyloleat oder Triglyceride, verwendet, oder wässrige Injektionssuspensionen, welche viskositätserhöhende Stoffe, z.B. Natrium-carboxymethylcellulose, Sorbit und/oder Dextran und gegebenenfalls Stabilisatoren enthalten.

Inhalationspräparate für die Behandlung der Atemwege durch nasale oder buccale Verabreichung sind z.B. Aerosole oder Sprays, welche den pharmakologischen Wirkstoff in Form eines Puders oder in Form von Tropfen einer Lösung oder Suspension verteilen können. Präparate mit Puder-verteilenden Eigenschaften enthalten ausser dem Wirkstoff üblicherweise ein flüssiges Treibgas mit einem Siedepunkt unter der Raumtemperatur, sowie, wenn erwünscht, Trägerstoffe, wie flüssige oder feste, nicht-ionische oder anionische oberflächenaktive Mittel und/oder feste Verdünnungsmittel. Präparate, in welchen der pharmakologische Wirkstoff in Lösung vorliegt, enthalten ausser diesen ein geeignetes Treibmittel, ferner, falls notwendig, ein zusätzliches Lösungsmittel und/oder einen Stabilisator. Anstelle des Treibgases kann auch Druckluft verwendet werden, wobei diese

mittels einer geeigneten Verdichtungs- und Entspannungsvorrichtung nach Bedarf erzeugt werden kann.

Pharmazeutische Präparate für topische und lokale Verwendung sind z.B. für die Hautbehandlung Lotionen und Crème , die eine flüssige oder halbfeste Oel-in-wasser- oder Wasser-in-Oel-Emulsion enthalten, und Salben, gegebenenfalls ein Konservierungsmittel enthaltend, für die Augen Augentropfen, welche die aktive Verbindung in wässriger oder oeliger Lösung enthalten und Augensalben, die vorzugsweise in steriler Form hergestellt werden, für die Behandlung der Nase Puder, Aerosole und Sprays (ähnlich den oben beschriebenen für die Behandlung der Atemwege), sowie grobe Puder, die durch schnelles Inhalieren durch die Nase verabreicht werden und Nasentropfen, welche die aktive Verbindung in wässriger oder oeliger Lösung enthalten, oder für die lokale Mundbehandlung Lutschbonbons, welche die aktive Verbindung in einem allgemeinen aus Zucker und arabischem Gummi oder Tragakant gebildeten Masse enthalten, welcher Geschmacksstoffe beigegeben sein können, sowie Pastillen, die den Aktivstoff in einer inerten Masse, z.B. aus Gelatine und Glycerin oder Zucker und arabischem Gummi enthalten.

Die Erfindung betrifft schliesslich die Verwendung von Verbindungen der Formel I oder ihrer Salze als pharmakologisch aktive Verbindungen, insbesondere als Antiallergika, vorzugsweise in Form von pharmazeutischen Präparaten. Die tägliche Dosis, die einem Warmblüter von etwa 70 kg verabreicht wird, beträgt, je nach Applikationsform, von etwa 100 mg bis etwa 1000 mg, vorzugsweise von etwa 250 mg bis etwa 750 mg.

Die nachfolgenden Beispiele illustrieren die vorstehend beschriebene Erfindung; sie sollen jedoch diese in ihrem Umfang in keiner Weise einschränken. Temperaturen sind in Celsiusgraden angegeben.

Beispiel 1

Eine Lösung von 24 g 1-[N-(8-Methoxychinolin-6-yl)]-2-amino—fumarsäuredimethylester in 300 ml Diphenyläther wird 10 Minuten zum Rückfluss erhitzt. Man lässt auf Raumtemperatur abkühlen, versetzt mit 200 ml Diäthyläther, filtriert ab und kristallisiert aus Dimethylformamid um. Man erhält den 6-Methoxy-1-oxo-1,4-dihydro-4,7-phenanthrolin-3-carbonsäuremethylester vom Smp. 280° (Zers.).

Das Ausgangsmaterial kann beispielsweise folgendermassen erhalten werden:

Eine Lösung von 26 g 6-Amino-8-methoxy-chinolin in 300 ml Methanol wird mit 24 g Acetylendicarbonsäuredimethylester versetzt, 1 Stunde bei Raumtemperatur stehen gelassen und unter vermindertem Druck zur Trockene eingedampft. Der Eindampfrückstand wird aus Aethanol umkristallisiert. Man erhält den 1-[N-(8-Methoxychinolin-6-yl)]-2-amino-fumarsäuredimethylester vom Smp. 155°.

Beispiel 2

5 g 6-Methoxy-1-oxo-1,4-dihydro-4,7-phenanthrolin-3-carbonsäuremethylester werden in 100 ml n-Natronlauge gelöst und 1 Stunde zum Rückfluss erhitzt. Man lässt auf Raumtemperatur abkühlen und säuert mit verdünnter Salzsäure auf pH= 5 an. Nach einigem Stehenlassen kristallisiert die 6-Methoxy-1-oxo-1,4-dihydro-4,7-phenanthrolin-3-carbonsäure vom Smp. 286-288° (Zers.) aus.

Beispiel 3

In analoger Weise wie in den Beispielen 1 und 2 beschrieben kann man ferner herstellen.

6-Methoxy-1-oxo-1,4-dihydro-4,7-phenanthrolin-3-carbonsäureäthylester, Smp. 255°,

6-Methoxy-1-oxo-1,4-dihydro-4,7-phenanthrolin-3-carbonsäurepropylester, Smp. 202-204°,

6-Methoxy-1-oxo-1,4-dihydro-4,7-phenanthrolin-3-carbonsäure-n-butylester, Smp. 212-213°,

1-Aethoxy-6-methoxy-4,7-phenanthrolin-3-carbonsäuremethylester, Smp. 188-190°, und

6-n-Butoxy-1-oxo-1,4-dihydro-4,7-phenanthrolin-3-carbonsäuremethylester.

Beispiel 4

150 ml absolutes n-Butanol werden unter Rühren mit 30 mg Natriumhydrid versetzt. Das Reaktionsgefäss wird mit einem Extraktionsaufsatz nach Soxhlet versehen und dieser mit 6 g 6-Methoxy-1-oxo-1,4-dihydro-4,7-phenanthrolin-carbonsäuremethylester beschickt. Dann wird bei einer Badtemperatur von 160-180° 13 Stunden lang unter Stickstoff extrahiert und die Extraktionslösung anschliessend unter vermindertem Druck zur Trockne eingedampft. Der Rückstand wird mit wenig Eiswasser versetzt und dreimal mit je 100 ml Chloroform ausgezogen. Die Auszüge werden vereinigt, über Natriumsulfat getrocknet und unter vermindertem Druck zur Trockne eingedampft. Man erhält 6-Methoxy-1-oxo-1,4-dihydro-4,7-phenanthrolin-3-carbonsäurebutylester, der nach Umkristallisieren aus einem Gemisch von Aethanol, Petroläther und Diäthyläther bei 212-213° schmilzt.

Beispiel 5

In analoger Weise wie im Beispiel 4 beschrieben erhält man durch Umsetzung von 4 g 6-Methoxy-1-oxo-1,4-dihydro-4,7-phenanthrolin-3-carbonsäuremethylester mit Propanol den 6-Methoxy-1-oxo-1,4-dihydro-4,7-phenanthrolin-3-carbonsäurepropylester vom Smp. 202-204° (aus Toluol).

Beispiel 6

Eine Lösung von 6,5 g 6-Methoxy-1-oxo-1,4-dihydro-4,7-phenanthrolin-3-carbonsäuremethylester in 50 ml absolutem Dimethylformamid wird unter Rühren bei 40° in einer Inertgasatmosphäre mit 1,2 g Natriumhydrid (50%-ige Suspension in Mineralöl) versetzt. Nach 30 Minuten lässt man auf Raumtemperatur abkühlen, fügt portionsweise 4,3 g Aethyljodid hinzu, lässt 6 Stunden nachrühren, versetzt mit 200 ml Eiswasser, schüttelt mit 50 ml Petroläther aus und extrahiert dann dreimal mit je 200 ml Chloroform. Die Chloroformauszüge werden vereinigt, mit Wasser gewaschen, über Natriumsulfat getrocknet und unter vermindertem Druck eingedampft. Man erhält 1-Aethoxy-6-methoxy-4,7-phenanthrolin-3-carbonsäuremethylester, der nach Umkristallisieren aus Aethanol bei 188-190° schmilzt.

Beispiel 7

Zu einer Lösung von 2,5 g N-(5-Acetyl-8-methoxy--chinolin-6-yl)-oxamidsäuremethylester in 50 ml wasserfreiem N,N-Dimethylformamid fügt man 2 g wasserfreies Kaliumtertiärbutanolat hinzu und erwärmt unter Wasserausschluss 10 Stunden auf 130°. Dann lässt man abkühlen und dampft im Vakuum zur Trockne ein. Den Eindampfrückstand behandelt man nacheinander mit 2n-Essigsäure und Wasser. Durch chromatographische Reinigung an Silikagel und fraktionierte Kristallisation aus

N,N-Dimethylformamid erhält man den 6-Methoxy-1-oxo-1,4-dihydro-4,7-phenanthrolin-3-carbonsäuremethylester vom F 280° (Zers.).

Das Ausgangsmaterial kann beispielsweise folgendermasse erhalten werden:
Eine Lösung von 4,3 g 5-Acetyl-6-amino-8-methoxy-chinolin in 100 ml wasserfreiem Pyridin wird unter Rühren bei 10° tropfnasse mit 2,6 g Chlorglyoxylsäuremethylester versetzt. Nach beendeter Zugabe lässt man 3 Stunden bei Raumtemperatur nachrühren und dampft man im Vakuum bei 40° zur Trockne ein. Den Eindampfrückstand versetzt man mit 50 ml gesättigter Natriumhydrogencarbonatlösung und extrahiert mehrmals mit Essigsäureäthylester und Chloroform. Die organischen Extrakte werden über Natriumsulfat getrocknet und im Vakuum eingedampft. Das so erhaltene rohe N-(5-Acetyl-8-methoxy-chinolin-6-yl)-oxamidmethylester wird direkt weiterverarbeitet.

Beispiel 8

Zu einer Lösung von 5,3 g rohem 4-(6-Nitro-8-methoxy-chinolin-5-yl)-2,4 dioxo-buttersäureäthylester in 200 ml Essigsäure fügt man 2 g Palladium (5%-ig auf Aktivkohle) und hydriert bis zur Aufnahme von 3 Aequivalenten Wasserstoff. Dann filtriert man heiss vom Katalysator ab, versetzt mit 2 ml konzentrierter Salzsäure und dampft zur Trockne ein. Den Eindampfrückstand behandelt man nacheinander mit gesättigter wässriger Natriumbicarbonatlösung und Wasser. Nach der chromatographischen Reinigung an Kieselgel erhält man durch fraktionierte Kristallisation aus Dimethylformamid/Aethanol den 6-Methoxy-1-oxo-1,4-dihydro-4,7-phenanthrolin-3-carbonsäureäthylester vom F. 255°.
Das Ausgangsmaterial kann beispielsweise folgendermasse erhalten werden:

Zu einer Lösung von 10 g 5-Acetyl-6-nitro-8-methoxy-chinolin in 200 ml Aethanol und 3 g Natriumäthanolat fügt man 30 ml Oxalsäurediäthylester und erhitzt unter Rühren 12 Stunden unter Wasserausschluss zum Rückfluss. Dann versetzt man mit 100 ml Toluol und erwärmt die Reaktionsmischung weitere 5 Stunden, dampft man im Vakuum zur Trockne ein und verreibt den Rückstand zuerst mit 2n-Essigsäure und dann mit Wasser. Der so erhaltene rohe 4-(6-Nitro-8-methoxy-chinolin-6-yl)-2,4-dioxo-buttersäureäthylester kann ohne weitere Reinigung weiterverarbeitet werden.

Beispiel 9

Zu einer Lösung von 6,2 g 1-Chlor-6-methoxy-4,7-phenanthrolin-3-carbonsäuremethylester in 100 ml wasserfreiem N,N-Dimethylformamid fügt man 2 g Natriumäthanolat hinzu und erwärmt unter Rühren und Wasserausschluss 10 Stunden auf 100°. Dann dampft man im Vakuum zur Trockne ein, verteilt den Eindampfrückstand zwischen 3-mal 200 ml Chloroform und 200 ml Wasser, trocknet die organischen Phasen über Natriumsulfat und dampft im Vakuum zur Trockne ein. Aus dem Eindampfrückstand erhält man durch fraktionierte Kristallisation aus Aethanol den 1-Aethoxy-6-methoxy-4,7-phenanthrolin-3-carbonsäuremethylester vom F. 188-190°.

Das Ausgangsmaterial kann beispielsweise folgendermasse erhalten werden:
Zu 10 g 1-[N-(8-Methoxy-chinolin-6-yl)]-2-amino-fumarsäuredimethylester fügt man 100 ml Phosphoroxychlorid hinzu und erhitzt 15 Minuten unter Wasserausschluss und Rühren zum Sieden. Dann dampft man im Vakuum zur Trockne ein, verteilt den Eindampfrückstand zwischen 3-mal 200 ml Chloroform und 200 ml gesättigter wässriger Natriumbicarbonatlösung. Die organischen Phasen werden über Natriumsulfat getrocknet und im Vakuum eingedampft. Der so erhaltene 1-Chlor-6-methoxy-4,7-phenanthrolin-3-carbonsäuremethylester wird ohne weitere Reinigung weiterverarbeitet.

Beispiel 10

8,4 g 1-Imino-6-methoxy-1,4-dihydro-4,7-phenanthrolin-3-carbonsäurenitril werden in 200 ml äthanolischer Salzsäure (6n) über Nacht zum Rückfluss erhitzt. Dann wird unter vermindertem Druck zur Trockne eingedampft, zweimal mit Wasser gewaschen und dreimal mit je 250 ml 2n-Natronlauge ausgekocht. Die Auszüge werden vereinigt, mit konzentrierter Salzsäure schwach angesäuert und zur Kristallisation gestellt. Man erhält die 6-Methoxy-1-oxo-1,4-dihydro-4,7-phenanthrolin-3-carbonsäure vom Smp. 286-288°.

Das Ausgangsmaterial kann beispielsweise folgendermasse erhalten werden:

16,2 g 6-Amino-8-methoxy-chinolin werden in 300 ml Aethanol gelöst, mit 8 g Acetylendicarbonsäuredinitril versetzt und 2 Stunden bei Raumtemperatur gerührt. Dann wird unter vermindertem Druck zur Trockne eingedampft und aus Aethanol umkristallisiert. Man erhält das 1-Imino-6-methoxy-1,4-dihydro-4,7-phenanthrolin-3-carbonsäurenitril, das ohne weitere Reinigung weiterausgesetzt werden kann.

Beispiel 11

Eine zur Inhalation geeignete 1%-ige wässrige Lösung von 6-Methoxy-1-oxo-1,4-dihydro-4,7-phenanthrolin-3-carbonsäure kann wie folgt hergestellt werden:

Zusammensetzung (für 100 ml)

6-Methoxy-1-oxo-1,4-dihydro-4,7-phenanthrolin-3-carbonsäure

| | | |
|---|---|---|
| Natrium-4-äthoxy-6-butyryl-7-methyl-chinolin-methylester | | 1,000 g |
| Dinatriumsalz der Aethylendiamintetraessigsäure ( Stabilisator) | | 0,010 g |
| Benzalkoniumchlorid (Konservierungsmittel) | | 0,010 g |
| Wasser, destilliert | ad | 100 ml |

Der 6-Methoxy-1-oxo-1,4-dihydro-4,7-phenanthrolin-3-carbonsäure wird unter Zusatz der äquivalenten Menge Natriumhydroxid in frisch destilliertem Wasser gelöst und die Lösung mit dem Dinatriumsalz der Aethylendiamintetraessigsäure und dem Benzalkoniumchlorid, das ein Gemisch von Alkyl-methyl-benzyl-ammoniumchloriden ist, worin Alkyl von 8-18 Kohlenstoffatome enthält, versetzt. Nach vollständiger Auflösung der Komponenten wird die erhaltene Lösung mit Wasser auf ein Volumen von 100 ml gebracht, abgefüllt und gasdicht verschlossen.

Beispiel 12

Zur Insufflation geeignete, 0,025 g 6-Methoxy-1-oxo-1,4-dihydro-4,7-phenanthrolin-3-carbonsäuremethylester enthaltende Kapseln können wie folgt hergestellt werden:

Zusammensetzung (für 1000 Kapseln)

| | |
|---|---|
| 6-Methoxy-1-oxo-1,4-dihydro-4,7-phenanthrolin-3-carbonsäuremethylester | 25,00 g |
| Laktose, gemahlen | 25,00 g |

Der 6-Methoxy-1-oxo-1,4-dihydro-4,7-phenanthrolin-3-carbonsäuremethylester und die Laktose (feinst gemahlen) werden gut miteinander vermischt. Das erhaltene Pulver wird gesiebt und in Portionen zu je 0,05 g in Gelatinekapseln abgefüllt.

Beispiel 13

Tabletten enthalten 100 mg 6-Methoxy-1-oxo-1,4-dihydro-4,7-phenanthrolin-3-carbonsäuremethylester als Wirkstoff können beispielsweise in folgender Zusammensetzung hergestellt werden:

| Zusammensetzung | pro Tablette |
|---|---|
| Wirkstoff | 100 mg |
| Milchzucker | 50 mg |
| Weizenstärke | 73 mg |
| Koloidale Kieselsäure | 13 mg |
| Talk | 12 mg |
| Magnesiumstearat | 2 mg |
| | 250 mg |

Herstellung

Der Wirkstoff wird mit dem Milchzucker, einem Teil der Weizenstärke und mit koloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist. Diese Masse wird durch ein Sieb von etwa 1 mm Maschenweite getrieben, getrocknet, und das trockene Granulat nochmals durch ein Sieb getrieben. Dann werden die restliche Weizenstärke, der Talk und das Magnesiumstearat zugemischt. Die erhaltene Tablettiermischung wird zu Tabletten von je 250 mg mit Bruchkerbe (n) verpresst.

Beispiel 14

In analoger Weise wie in den Beispielen 11 bis 13 beschrieben können auch pharmazeutische Präparate enthaltend 6-Methoxy-1-oxo-1,4-dihydro-4,7-phenanthrolin-3-carbonsäure, 6-Methoxy-1-oxo-1,4-dihydro-4,7-phenanthrolin-3-carbonsäureäthylester,

6-Methoxy-1-oxo-1,4-dihydro-4,7-phenanthrolin-3-carbonsäure-propylester,

6-Methoxy-1-oxo-1,4-dihydro-4,7-phenanthrolin-3-carbonsäure-n-butylester,

1-Aethoxy-6-methoxy-4,7-phenanthrolin-3-carbonsäuremethylester und

6-n-Butoxy-1-oxo-1,4-dihydro-4,7-phenanthrolin-3-carbonsäuremethylester, gegebenenfalls in Form von Säure-additionssalzen, insbesondere der Hydrochloride, Carbon-säuren ferner in Form der Natriumsalze, hergestellt werden.

Beispiel 15: In analoger Weise wie in den Beispielen 1 bis 10 beschrieben kann man ferner
6-Butoxy-1-oxo-1,4-dihydro-4,7-phenanthrolin-3-carbonsäure-butylester sowie
4-Aethyl-6-methoxy-1-oxo-1,4-dihydro-4,7-phenanthrolin-3-carbonsäurebutylester
herstellen.

Patentansprüche:
(für alle benannten Länder ausser Oesterreich)

1. Neue 4,7-Phenanthrolinderivate der Formel (I),

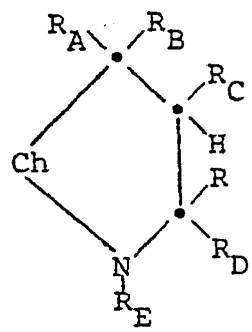

(I),

worin Ch einen gegebenenfalls substituierten 5,6-Chinolinylenrest bedeutet, dessen 6-Stellung mit der Gruppe $-N(R_E)-$ verbunden ist, R eine gegebenenfalls veresterte oder amidierte Carboxygruppe bedeutet, und worin entweder $R_A$ und $R_B$ gemeinsam Oxo darstellen, $R_C$ und $R_D$ gemeinsam eine zusätzliche Bindung bedeuten und $R_E$ einen Rest $R_1$ darstellt, der Wasserstoff oder einen aliphatischen, cycloaliphatischen, araliphatischen oder heterocyclisch-aliphatischen Rest bedeutet, oder $R_A$ eine gegebenenfalls verätherte Hydroxygruppe bedeutet und $R_B$ gemeinsam mit $R_C$ sowie $R_D$ gemeinsam mit $R_E$ jeweils eine zusätzliche Bindung darstellen, und Salze von salzbildenden Verbindungen der Formel (I).

2. Verbindungen gemäss Anspruch 1 der Formel (I), worin Ch gegebenenfalls zusätzlich durch Niederalkyl, Niederalkoxy, Hydroxy und/oder Halogen substituiertes 5,6-Chinolinylen bedeutet, R Carboxy, Niederalkoxycarbonyl, Hydroxyniederalkoxycarbonyl, Niederalkoxyniederalkoxycarbonyl, Diniederalkylaminoniederalkoxy- oder 5-7-gliedriges Niederalkylenamino- bzw. Aza-, Oxa- oder Thianiederalkylenaminoniederalkoxycarbonyl oder amidiertes Carboxy bedeutet, das als Aminogruppe Amino, Hydroxyamino,

Niederalkylamino, Diniederalkylamino oder 5-7-gliedriges Niederalkylen- bzw. Aza-, Oxa-, Thianiederalkylenamino aufweist, und worin entweder $R_A$ und $R_B$ gemeinsam Oxo darstellen, $R_C$ und $R_D$ gemeinsam eine zusätzliche Bindung bedeuten und $R_E$ Wasserstoff, Niederalkyl, Hydroxyniederalkyl, Niederalkoxyniederalkyl, Diniederalkylaminoniederalkyl, 5-7-gliedriges Niederalkylenamino- bzw. Aza-, Oxa- oder Thianiederalkylenaminoniederalkyl, Niederalkenyl, Cycloalkyl mit 3-8 Ringgliedern, gegebenenfalls durch Niederalkyl, Niederalkoxy und/oder Halogen substituiertes Phenylniederalkyl, Furylniederalkyl, Thienylniederalkyl oder Pyridylniederalkyl darstellt, oder $R_A$ Hydroxy, Niederalkoxy oder Niederalkenyloxy bedeutet und $R_B$ gemeinsam mit $R_C$ sowie $R_D$ gemeinsam mit $R_E$ jeweils eine zusätzliche Bindung darstellen, und Salze von vorstehend definierten Verbindungen mit salzbildenden Eigenschaften.

3. Verbindungen gemäss Anspruch 1 der Formel (Ia) oder (Ib)

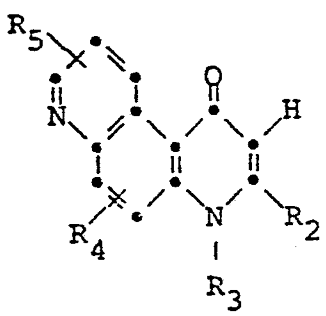

, (Ia)

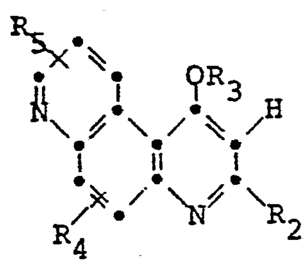

, (Ib)

worin $R_2$ für Carboxy, Niederalkoxycarbonyl mit bis zu 5 Kohlenstoffatomen, Hydroxyniederalkoxycarbonyl mit bis zu 5 Kohlenstoffatomen, Niederalkoxyniederalkoxycarbonyl mit bis zu 4 Kohlenstoffatomen in den Alkoxyteilen, Diniederalkylaminoniederalkoxycarbonyl mit jeweils bis zu 4 Kohlenstoffatomen im Alkyl- und Alkoxyteil, Carbamyl, N-Hydroxycarbamyl oder N-Niederalkyl- bzw. N,N-Diniederalkylcarb-

amyl mit bis zu 4 Kohlenstoffatomen im Alkyl steht, $R_3$ Wasserstoff, Niederalkyl mit bis zu 4 Kohlenstoffatomen, Niederalkenyl mit bis zu 4 Kohlenstoffatomen, gegebenenfalls durch Niederalkyl mit bis zu 4 Kohlenstoffatomen, Niederalkoxy mit bis zu 4 Kohlenstoffatomen und/oder Halogen bis und mit Atomnummer 35 substituiertes Phenyl-, Furyl-, Thienyl- oder Pyridylniederalkyl mit bis zu 4 Kohlenstoffatomen im Alkylteil, Niederalkoxyniederalkyl mit bis zu 4 Kohlenstoffatomen im Alkoxy- bzw. Alkylteil oder Diniederalkylaminoniederalkyl bzw. Niederalkylen- oder Aza-, Thia-, oder Oxaniederalkylenaminoniederalkyl, worin Niederalkyl bis zu 4 Kohlenstoffatomen und Niederalkylen bzw. Aza-, Oxa- oder Thianiederalkylen 5 - 7 Ringglieder aufweist, bedeutet, und $R_4$ und $R_5$ unabhängig voneinander Wasserstoff, Niederalkyl mit bis zu 4 Kohlenstoffatomen, Niederalkoxy mit bis zu 4 Kohlenstoffatomen, Hydroxy oder Halogen bis einschliesslich Atomnummer 35 bedeuten, und Salze von salzbildenden Verbindungen der Formel (Ia) oder (Ib).

4. Verbindungen gemäss Anspruch 1 der Formel (II)

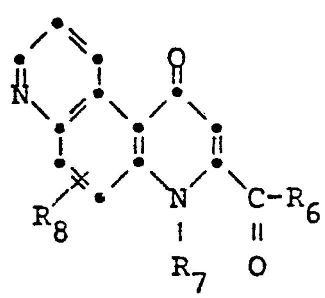

(II) ,

worin $R_6$ Hydroxy oder Niederalkoxy mit bis zu 4 Kohlenstoffatomen bedeutet, $R_7$ Wasserstoff oder Niederalkyl mit bis zu 4 Kohlenstoffatomen bedeutet, und $R_8$ Wasserstoff oder Niederalkoxy mit bis zu 4 Kohlenstoffatomen bedeutet, und Salze von salzbildenden Verbindungen der Formel (II).

5. 6-Methoxy-1-oxo-1,4-dihydro-4,7-phenanthrolin-3-carbonsäuremethylester.

6. 6-Methoxy-1-oxo-1,4-dihydro-4,7-phenanthrolin-3-carbonsäure.

7. 6-Methoxy-1-oxo-1,4-dihydro-4,7-phenanthrolin-3-carbonsäureäthylester,
6-Methoxy-1-oxo-1,4-dihydro-4,7-phenanthrolin-3-carbonsäurepropylester,
6-Methoxy-1-oxo-1,4-dihydro-4,7-phenanthrolin-3-carbonsäure-n-butylester,
1-Aethoxy-6-methoxy-4,7-phenanthrolin-3-carbonsäuremethylester oder
6-n-Butoxy-1-oxo-1,4-dihydro-4,7-phenanthrolin-3-carbonsäuremethylester.

8. Pharmazeutische Präprate enthaltend eine Verbindung gemäss einem der Ansprüche 1 - 7 oder ein pharmazeutisch verwendbares Salz einer Verbindung gemäss einem der Ansprüche 1 - 7 neben üblichen pharmazeutischen Hilfs- und Trägerstoffen.

9. Verfahren zur Herstellung neuer 4,7-Phenanthrolinderivate der Formel

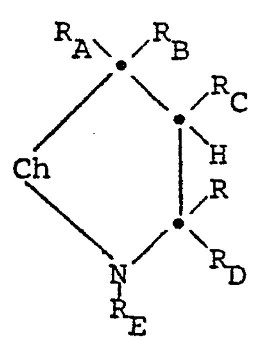

(I),

worin Ch einen gegebenenfalls substituierten 5,6-Chinolinylenrest bedeutet, dessen 6-Stellung mit der Gruppe $-N(R_E)-$ verbunden ist, R eine gegebenenfalls veresterte oder amidierte Carboxygruppe bedeutet, und worin entweder $R_A$ und $R_B$ gemeinsam Oxo darstellen, $R_C$ und $R_D$ gemeinsam eine zusätzliche Bindung bedeuten und $R_E$ einen Rest $R_1$ darstellt, der Wasserstoff oder einen aliphatischen, cycloaliphatischen, araliphatischen oder heterocyclisch-aliphatischen Rest bedeutet, oder $R_A$ eine gegebenenfalls verätherte Hydroxygruppe bedeutet und $R_B$ gemeinsam mit $R_C$ sowie $R_D$ gemeinsam mit $R_E$ jeweils eine zusätzliche Bindung darstellen, und von Salzen der salzbildenden Verbindungen der Formel (I), dadurch gekennzeichnet, dass man eine Verbindung der Formel (IV)

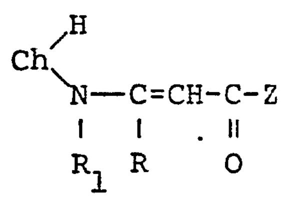

(IV) ,

worin Z einen abspaltbaren Rest bedeutet, oder eine Verbindung der Formel (VI)

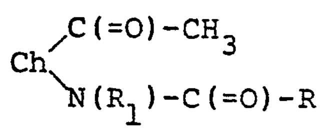

(VI)

oder eine Verbindung der Formel (VIII)

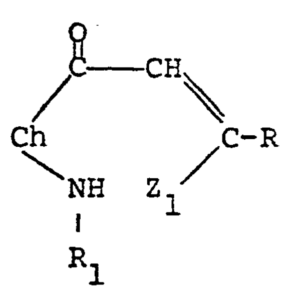

(VIII) ,

worin $Z_1$ eine gegebenenfalls reaktionsfähige abgewandelte Hydroxygruppe bedeutet, oder ein Tautomeres und/oder Salz davon intramolekular cyclisiert, oder in einer Verbindung der Formel (XI)

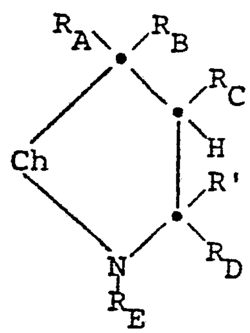

(XI) ,

worin der Rest R' einen in die gegebenenfalls veresterte oder amidierte Carboxygruppe R überführbaren Rest bedeutet, oder in einem Salz davon die Gruppe R' in die gegebenenfalls veresterte oder amidierte Carboxygruppe R überführt, oder in einer Verbindung der Formel (XIII)

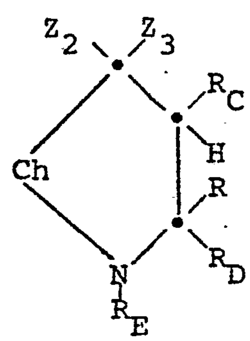

(XIII) ,

worin $Z_2$ ein in gegebenenfalls veräthertes Hydroxy überführbaren Rest bedeutet und $Z_3$ gemeinsam mit $R_C$ sowie $R_D$ gemeinsam mit $R_E$ jeweils eine zusätzliche Bindung darstellt, oder worin $Z_2$ und $Z_3$ jeweils einen einwertigen oder gemeinsamen einen zweiwertigen in Oxo überführbaren Rest bedeuten, und $R_C$ in gemeinsam mit $R_D$ eine zusätzliche Bindung darstellt, oder in einem Salz davon einen in gegebenenfalls veräthertes Hydroxy überführbaren Rest $Z_2$

in gegebenenfalls veräthertes Hydroxy bzw. in Oxo überführbare Reste $Z_2$ und $Z_3$ oder $Z_2 + Z_3$ in Oxo überführt, oder in einer Verbindung der Formel XVI

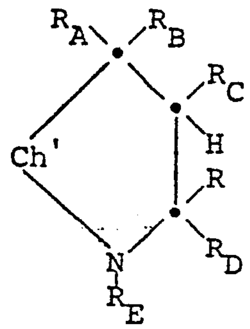

(XVI) ,

worin Ch' einen partiell hydrierten Rest Ch bedeutet, oder in einem Salz davon den Rest Ch' zu Ch oxydiert, oder aus einer Verbindung der Formel XVIII

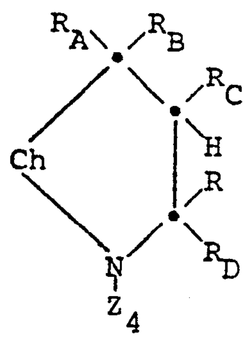

(XVIII) ,

worin $Z_4$ einen abspaltbaren Rest bedeutet, oder aus einem Salz davon $Z_4$ abspaltet, und gewünschtenfalls die so erhältliche Verbindung in eine andere Verbindung der Formel I und/oder eine erhaltene salzbildende Verbindung in ein Salz oder ein erhaltenes Salz in die freie Verbindung oder in ein anderes Salz überführt.

10. Neue 4,7-Phenanthrolinderivate der Formel (I),

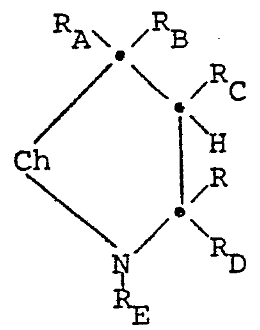

(I),

worin Ch einen gegebenenfalls substituierten 5,6-Chinolinylenrest bedeutet, dessen 6-Stellung mit der Gruppe $-N(R_E)-$ verbunden ist, R eine gegebenenfalls veresterte oder amidierte Carboxygruppe bedeutet, und worin entweder $R_A$ und $R_B$ gemeinsam Oxo darstellen, $R_C$ und $R_D$ gemeinsam eine zusätzliche Bindung bedeuten und $R_E$ einen Rest $R_1$ darstellt, der Wasserstoff oder einen aliphatischen, cycloaliphatischen, araliphatischen oder heterocyclisch-aliphatischen Rest bedeutet, oder $R_A$ eine gegebenenfalls verätherte Hydroxygruppe bedeutet und $R_B$ gemeinsam mit $R_C$ sowie $R_D$ gemeinsam mit $R_E$ jeweils eine zusätzliche Bindung darstellen, und pharmazeutisch verwendbare Salze von salzbildenden Verbindungen der Formel (I) zur Anwendung in einem Verfahren zur prophylaktischen und/oder therapeutischen Behandlung des menschlichen oder tierischen Körpers.

Patentansprüche:
(für Oesterreich)

1. Verfahren zur Herstellung neuer 4,7-Phenanthrolinderivate der Formel

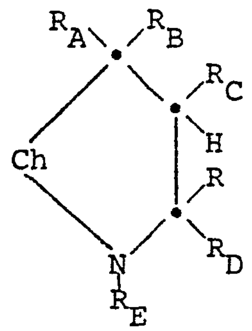

, (I)

worin Ch einen gegebenenfalls substituierten 5,6-Chinolinylenrest bedeutet, dessen 6-Stellung mit der Gruppe $-N(R_E)-$ verbunden ist, R eine gegebenenfalls veresterte oder amidierte Carboxygruppe bedeutet, und worin entweder $R_A$ und $R_B$ gemeinsam Oxo darstellen, $R_C$ und $R_D$ gemeinsam eine zusätzliche Bindung bedeuten und $R_E$ einen Rest $R_1$ darstellt, der Wasserstoff oder einen aliphatischen, cycloaliphatischen, araliphatischen oder heterocyclisch-aliphatischen Rest bedeutet, oder $R_A$ eine gegebenenfalls verätherte Hydroxygruppe bedeutet und $R_B$ gemeinsam mit $R_C$ sowie $R_D$ gemeinsam mit $R_E$ jeweils eine zusätzliche Bindung darstellen, und von Salzen der salzbildenden Verbindungen der Formel (I), dadurch gekennzeichnet, dass man eine Verbindung der Formel (IV)

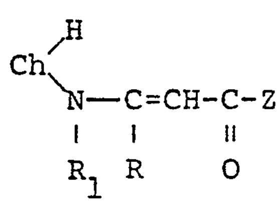

, (IV)

worin Z einen abspaltbaren Rest bedeutet, oder eine Verbindung der Formel (VI)

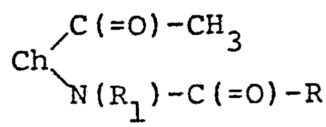

(VI)

oder eine Verbindung der Formel (VIII)

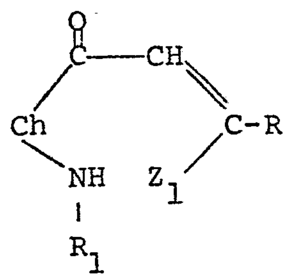

, (VIII)

worin $Z_1$ eine gegebenenfalls reaktionsfähige abgewandelte Hydroxygruppe bedeutet, oder ein Tautomeres und/oder Salz davon intramolekular cyclisiert, oder in einer Verbindung der Formel (XI)

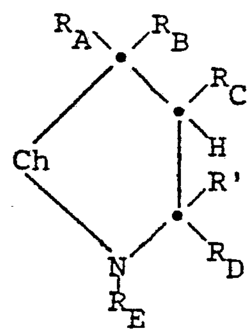

, (XI)

worin der Rest R' einen in die gegebenenfalls veresterte oder amidierte Carboxygruppe R überführbaren Rest bedeutet, oder in einem Salz davon die Gruppe R' in die gegebenenfalls veresterte oder amidierte Carboxygruppe R überführt, oder in einer Verbindung der Formel (XIII)

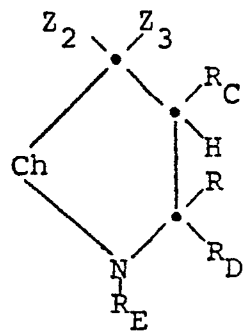

, (XIII)

worin $Z_2$ ein in gegebenenfalls veräthertes Hydroxy überführbaren Rest bedeutet und $Z_3$ gemeinsam mit $R_C$ sowie $R_D$ gemeinsam mit $R_E$ jeweils eine zusätzliche Bindung darstellt, oder worin $Z_2$ und $Z_3$ jeweils einen einwertigen oder gemeinsamen einen zweiwertigen in Oxo überführbaren Rest bedeuten, und $R_C$ in gemeinsam mit $R_D$ eine zusätzliche Bindung darstellt, oder in einem Salz davon einen in gegebenenfalls veräthertes Hydroxy überführbaren Rest $Z_2$ in gegebenenfalls veräthertes Hydroxy bzw. in Oxo überführbare Reste $Z_2$ und $Z_3$ oder $Z_2 + Z_3$ in Oxo überführt, oder in einer Verbindung der Formel XVI

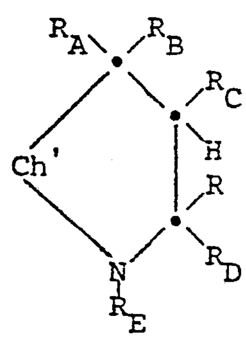

, (XVI)

worin Ch' einen partiell hydrierten Rest Ch bedeutet, oder in einem Salz davon den Rest Ch' zu Ch oxydiert, oder aus einer Verbindung der Formel XVIII

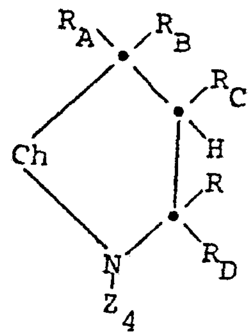

, (XVIII)

worin $Z_4$ einen abspaltbaren Rest bedeutet, oder aus einem Salz davon $Z_4$ abspaltet, und gewünschtenfalls die so erhältliche Verbindung in eine andere Verbindung der Formel I und/oder eine erhaltene salzbildende Verbindung in ein Salz oder ein erhaltenes Salz in die freie Verbindung oder in ein anderes Salz überführt.

2. Verfahren nach Anspruch 1, dadurch gekennzeichnet, dass man von einer Verbindung der Formel IV, worin Ch, R und $R_1$ die in Anspruch 1 angegebenen Bedeutungen haben und Z gegebenenfalls veräthertes oder mit einer anorganischen Säure verestertes Hydroxy bedeutet, oder einem Salz davon ausgeht.

3. Verfahren nach Anspruch 1, dadurch gekennzeichnet, dass man von einer Verbindung der Formel XI ausgeht, worin R' von verestertem oder amidiertem Carboxy R verschiedenes funktionell abgewandeltes Carboxy, insbesondere Cyano, bedeutet, Ch die in Anspruch 1 angegebene Bedeutung hat, $R_A$ und $R_B$ gemeinsam Oxo, $R_C$ und $R_D$ gemeinsam eine zusätzliche Bindung und $R_E$ Wasserstoff bedeuten, oder einem Salz davon ausgeht und R' zu gegebenenfalls verestertem oder amidiertem Carboxy solvolysiert insbesondere eine Cyanogruppe R' zu Carboxy oder Carbamyl hydrolysiert oder durch säurekatalysierte Umsetzung mit ein Alkohol in eine Iminoäthergruppe überführt und diese zu

verestertem Carboxy hydrolysiert.

4. Verfahren nach einem der Ansprüche 1 bis 3, dadurch gekennzeichnet, dass man Verbindungen gemäss Anspruch 1 der Formel (I), worin Ch gegebenenfalls zusätzlich durch Niederalkyl, Niederalkoxy, Hydroxy und/oder Halogen substituiertes 5,6-Chinolinylen bedeutet, R Carboxy, Niederalkoxycarbonyl, Hydroxyniederalkoxycarbonyl, Niederalkoxyniederalkoxycarbonyl, Diniederalkylaminoniederalkoxy- oder 5-7-gliedriges Niederalkylenamino- bzw. Aza-, Oxa- oder Thianiederalkylenaminoniederalkoxycarbonyl oder amidiertes Carboxy bedeutet, das als Aminogruppe Amino, Hydroxyamino, Niederalkylamino, Diniederalkylamino oder 5-7-gliedriges Niederalkylen- bzw. Aza-, Oxa-, Thianiederalkylenamino aufweist, und worin entweder $R_A$ und $R_B$ gemeinsam Oxo darstellen, $R_C$ und $R_D$ gemeinsam eine zusätzliche Bindung bedeuten und $R_E$ Wasserstoff, Niederalkyl, Hydroxyniederalkyl, Niederalkoxyniederalkyl, Diniederalkylaminoniederalkyl, 5-7-gliedriges Niederalkylenamino- bzw. Aza-, Oxa- oder Thianiederalkylenaminoniederalkyl, Niederalkenyl, Cycloalkyl mit 3 - 8 Ringgliedern, gegebenenfalls durch Niederalkyl, Niederalkoxy und/oder Halogen substistuiertes Phenylniederalkyl, Furylniederalkyl, Thienylniederalkyl oder Pyridylniederalkyl darstellt, oder $R_A$ Hydroxy, Niederalkoxy oder Niederalkenyloxy bedeutet und $R_B$ gemeinsam mit $R_C$ sowie $R_D$ gemeinsam mit $R_E$ jeweils eine zusätzliche Bindung darstellen, und Salze von vorstehend definierten Verbindungen mit salzbildenden Eigenschaften herstellt.

5. Verfahren nach einem der Ansprüche 1 bis 3, dadurch gekennzeichnet, dass man Verbindungen gemäss Anspruch 1 der Formel (Ia) oder (Ib)

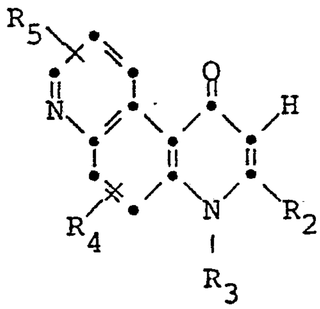, (Ia)

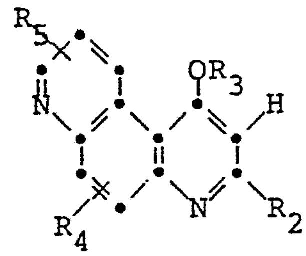, (Ib)

worin $R_2$ für Carboxy, Niederalkoxycarbonyl mit bis zu 5 Kohlenstoffatomen, Hydroxyniederalkoxycarbonyl mit bis zu 5 Kohlenstoffatomen, Niederalkoxyniederalkoxycarbonyl mit bis zu 4 Kohlenstoffatomen in den Alkoxyteilen, Diniederalkylaminoniederalkoxycarbonyl mit jeweils bis zu 4 Kohlenstoffatomen im Alkyl- und Alkoxyteil, Carbamyl, N-Hydroxycarbamyl oder N-Niederalkyl- bzw. N,N-Diniederalkylcarbamyl mit bis zu 4 Kohlenstoffatomen im Alkyl steht, $R_3$ Wasserstoff, Niederalkyl mit bis zu 4 Kohlenstoffatomen, Niederalkenyl mit bis zu 4 Kohlenstoffatomen, gegebenenfalls durch Niederalkyl mit bis zu 4 Kohlenstoffatomen, Niederalkoxy mit bis zu 4 Kohlenstoffatomen und/oder Halogen bis und mit Atomnummer 35 substituiertes Phenyl-, Furyl-, Thienyl- oder Pyridylniederalkyl mit bis zu 4 Kohlenstoffatomen im Alkylteil, Niederalkoxyniederalkyl mit bis zu 4 Kohlenstoffatomen im Alkoxy- bzw. Alkylteil oder Diniederalkylaminoniederalkyl bzw. Niederalkylen- oder Aza-, Thia-, oder Oxaniederalkylenaminoniederalkyl, worin Niederalkyl bis zu 4 Kohlenstoffatomen und Niederalkylen bzw. Aza-, Oxa- oder Thianiederalkylen 5 - 7 Ringglieder aufweist, bedeutet, und $R_4$ und $R_5$ unabhängig voneinander Wasserstoff, Niederalkyl mit bis zu

4 Kohlenstoffatomen, Niederalkoxy mit bis zu 4 Kohlenstoffatomen, Hydroxy oder Halogen bis einschliesslich Atomnummer 35 bedeuten, und Salze von salzbildenden Verbindungen der Formel (Ia) oder (Ib) herstellt.

6. Verfahren nach einem der Ansprüche 1 bis 3, dadurch gekennzeichnet, dass man Verbindungen gemäss Anspruch 1 der Formel (II)

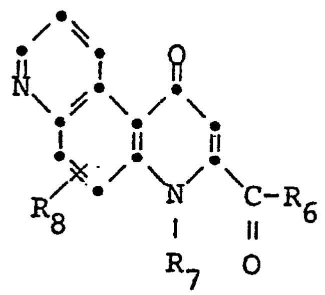

(II) ,

worin $R_6$ Hydroxy oder Niederalkoxy mit bis zu 4 Kohlenstoffatomen bedeutet, $R_7$ Wasserstoff oder Niederalkyl mit bis zu 4 Kohlenstoffatomen bedeutet, und $R_8$ Wasserstoff oder Niederalkoxy mit bis zu 4 Kohlenstoffatomen bedeutet und Salze von salzbildenden Verbindungen der Formel (II) herstellt.

7. Verfahren nach einem der Ansprüche 1 bis 3, dadurch gekennzeichnet, dass man Verbindungen gemäss Anspruch 1 der Formel (III)

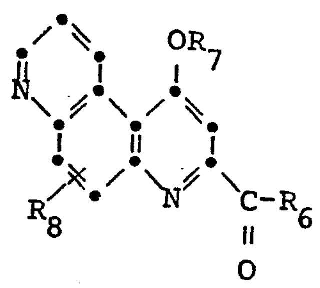

(III) ,

worin $R_6$ Hydroxy oder Niederalkoxy mit bis zu 4 Kohlenstoffatomen bedeutet, $R_7$ Wasserstoff oder Niederalkyl mit bis zu 4 Kohlenstoffatomen bedeutet, und $R_u$ Wasserstoff oder Niederalkoxy mit bis zu 4 Kohlenstoffatomen bedeutet, und Salze von salzbildenden Verbindungen der Formel (III) herstellt.

8. Verfahren nach einem der Ansprüche 1 bis 3, dadurch gekennzeichnet, dass man den 6-Methoxy-1-oxo-1,4-dihydro-4,7-phenanthrolin-3-carbonsäuremethylester herstellt.

9. Verfahren nach einem der Ansprüche 1 bis 3, dadurch gekennzeichnet, dass man den 6-Methoxy-1-oxo-1,4-dihydro-4,7-phenanthrolin-3-carbonsäureäthylester,
6-Methoxy-1-oxo-1,4-dihydro-4,7-phenanthrolin-3-carbonsäurepropylester,
6-Methoxy-1-oxo-1,4-dihydro-4,7-phenanthrolin-3-carbonsäure-n-butylester,
1-Aethoxy-6-methoxy-4,7-phenanthrolin-3-carbonsäuremethylester oder den
6-n-Butoxy-1-oxo-1,4-dihydro-4,7-phenanthrolin-3-carbonsäuremethylester herstellt.

10. Verfahren zur Herstellung von pharmazeutischen Präparaten enthaltend eine Verbindung gemäss einem der Ansprüche 1 und 4 - 9 oder ein pharmazeutisch verwendbares Salz davon neben üblichen pharmazeutischen Hilfs- und Trägerstoffen.

0013666

Europäisches Patentamt

# EUROPÄISCHER RECHERCHENBERICHT

Nummer der Anmeldung
EP 80 81 0010

| EINSCHLÄGIGE DOKUMENTE | | | KLASSIFIKATION DER ANMELDUNG (Int.Cl. [3]) |
|---|---|---|---|
| Kategorie | Kennzeichnung des Dokuments mit Angabe, soweit erforderlich, der maßgeblichen Teile | betrifft Anspruch | |
| | DE - A - 2 554 772 (FISONS)<br>* Ansprüche *<br>-- | 1,8 | C 07 D 471/04<br>A 61 K 31/47//<br>C 07 D 215/26<br>215/38<br>(C 07 D 471/04<br>221/00<br>221/00) |
| | DE - A - 2 149 692 (I.C.I.)<br>* Ansprüche *<br>---- | 1,8 | |

RECHERCHIERTE SACHGEBIETE (Int. Cl. [3])

C 07 D 471/04
A 61 K 31/47

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 16-04-1980 | ALFARO |